# EUROPÄISCHE PATENTSCHRIFT

(11) **EP 0 676 461 B1**
(45) Veröffentlichungstag und Bekanntmachung des Hinweises auf die Patenterteilung: **14.08.2002**
(21) Anmeldenummer: 95104475.9
(22) Anmeldetag: 27.03.1995
(51) Int. Cl.: C09K 11/06, C07D 413/04, C07D 263/32, C07D 263/56, C07D 307/79, C07D 307/80, C07D 307/81, C07D 271/10, C07C 13/72, H05B 33/14

(54) **Spiroverbindungen und ihre Verwendung als Elektrolumineszenzmaterialien**
Spiro compounds and their application as electroluminescence materials
Composés spiro et leur application comme matières électroluminescentes

(30) Priorität: 07.04.1994 DE 4411969; 25.11.1994 DE 4442063; 27.12.1994 DE 4446818
(43) Veröffentlichungstag der Anmeldung: 11.10.1995
(73) Patentinhaber: Covion Organic Semiconductors GmbH, 65926 Frankfurt (DE)
(72) Erfinder: Lupo, Donald, Dr., D-60316 Frankfurt (DE); Salbeck, Josef, Dr., D-65779 Kelkheim (DE); Schenk, Hermann, Dr., D-65719 Hofheim (DE); Stehlin, Thomas, Dr., D-65830 Kriftel (DE); Stern, Roland, Dr., D-65189 Wiesbaden (DE); Wolf, Arno, Dr., D-55131 Mainz (DE); Kreuder, Willi, Dr., D-55126 Mainz (DE)
(74) Vertreter: Luderschmidt, Schüler & Partner GbR

(56) Entgegenhaltungen:
- EP-A- 0 120 673
- EP-A- 0 502 202
- EP-A- 0 510 541
- DE-A- 4 331 401
- US-A- 5 026 894
- US-A- 5 151 629
- JOURNAL OF THE AMERICAN CHEMICAL SOCIETY, Bd. 112, 1990, Seiten 5662-3, XP002030896 J. M. TOUR ET AL.: "Approaches to Orthogonally Fused Conducting Polymers for Molecular Electronics"
- POLYMER PREPRINTS, Bd. 31, Nr. 1, April 1990, Seiten 408-9, XP002030897 J. M. TOUR ET AL.: "Spiro-Fused Conducting Polymers for Molecular Electronics"
- JOURNAL OF THE SOCIETY OF DYERS AND COLOURISTS, Bd. 94, Nr. 7, Juli 1978, Seiten 306-9, XP002030898 F. K. SUTCLIFFE ET AL.: "The Synthesis and Properties of Dyes and Pigments Containing a 9,9'-Spirobifluorene Residue"
- LIEBIGS ANNALEN DER CHEMIE, Nr. 6, 1981, Seiten 1118-38, XP002030899 B. LIPHARDT ET AL.: "Laserfarbstoffe, I. Bifluorophore Laserfarbstoffe zur Steigerung des Wirkungsgrades von Farbstoff-Lasern"
- HELVETICA CHIMICA ACTA, Bd. 52, Nr. 5, 1969, Seiten 1202-18, XP002030900 G. HAAS ET AL.: "Optisch aktive 9,9'-Spirobifluoren-Derivate"
- HELVETICA CHIMICA ACTA, Bd. 62, Nr. 7, 1979, Seiten 2285-2302, XP002030901 V. PRELOG ET AL.: "Chirale 2,2'-Polyoxaalkano-9,9'-spirobifluorene"
- TETRAHEDRON LETTERS, Bd. 32, Nr. 39, 1991, Seiten 5309-12, XP002030902 V. ALCAZAR MONTERO ET AL.: "Selective alpha,omega-dicarboxylic acid recognition in a chiral cleft shaped by the 9,9'-spirobifluorene unit"
- ANGEWANDTE CHEMIE, Bd. 104, Nr. 11, 1992, Seiten 1503-5, XP002030903 V. ALCAZAR ET AL.: "Enantioselektive Komplexierung chiraler Dicarbonsäuren in funktionalisierten spaltenförmigen 9,9'-Spirobifluorenen"

## Beschreibung

Es besteht ein hoher industrieller Bedarf an großflächigen Festkörper-Lichtquellen für eine Reihe von Anwendungen, überwiegend im Bereich von Anzeigeelementen, der Bildschirmtechnologie und der Beleuchtungstechnik. Die an diese Lichtquellen gestellten Anforderungen können zur Zeit von keiner der bestehenden Technologien völlig befriedigend gelöst werden.

Als Alternative zu herkömmlichen Anzeigeelementen, wie Glühlampen, Gasentladungslampen und nicht selbstleuchtenden Flüssigkristallanzeigeelementen, sind bereits seit einiger Zeit Elektrolumineszenz(EL)materialien und -vorrichtungen, wie lichtemittierende Dioden (LED), bekannt.

Elektrolumineszenzmaterialien sind Stoffe, die befähigt sind, beim Anlegen eines elektrischen Feldes Licht abzustrahlen. Das physikalische Modell zur Beschreibung dieses Effektes basiert auf der strahlenden Rekombination von Elektronen und Elektronenlücken ("Löchern"). Bei lichtemittierenden Dioden werden die Ladungsträger über die Kathode bzw. Anode in das Elektrolumineszenzmaterial injiziert.
Elektrolumineszenzvorrichtungen enthalten ein Lumineszenzmaterial als lichtemitterende Schicht.
Allgemein sind Elektrolumineszenzmaterialien und -vorrichtungen beispielsweise beschrieben in Ullmann's Encyclopedia of Industrial Chemistry, Vol A9, 5th Ed. VCH Verlag 1987 und der dort zitierten Literatur.
Neben anorganischen Stoffen, wie ZnS/Mn oder GaAs, sind auch organische Verbindungen als EL-Materialien bekannt geworden.

Eine Beschreibung von EL-Vorrichtungen, die niedermolekulare organischen EL-Materialien enthalten, findet sich beispielsweise in US 4,539,507.
Nachteile dieser niedermolekularen organischen Materialien sind beispielsweise die ungenügenden Filmbildungseigenschaften und eine ausgeprägte Tendenz zur Kristallisation.

In jüngerer Zeit sind auch Polymere als EL-Materialien beschrieben worden (siehe z.B. WO-A 90/13148). Jedoch ist die Lichtausbeute (Quanteneffizienz) bei diesen Stoffen beträchtlich geringer als bei den niedermolekularen Verbindungen.

Wünschenswert war es, EL-Materialien zu finden, die gute Lichtausbeuten zeigen und gleichzeitig zu dünnen homogenen Filmen verarbeitbar sind, die eine geringe Kristallisationsneigung aufweisen.

Es wurde nun überraschend gefunden, daß sich Spiroverbindungen, insbesondere Derivate des 9,9'-Spirobifluorens, in hervorragender Weise als EL-Materialien eignen.

Einzelne Verbindungen dieser Art sind beispielsweise in US-A 5,026,894, J. M. Tour et al. J. Am. Chem. Soc. 112 (1990) 5662 und J. M. Tour et al. Polym. Prepr. (1990) 408 als Verknüpfungselemente für polymere, organische Halbleiter beschrieben und als Materialien für molekulare Elektronik vorgeschlagen. Über eine mögliche Verwendung als EL-Materialien wird jedoch nichts gesagt.

Gegenstand der Erfindung ist daher die Verwendung von Spiroverbindungen der allgemeinen Formel (II), 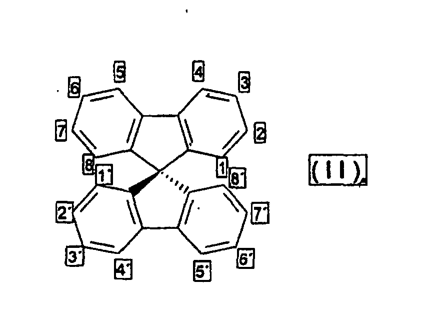 wobei die Benzogruppen unabhängig voneinander substituiert und/oder anelliert sein können.

Verbindungen der Formel (II) zeichnen sich durch eine gute Löslichkeit in gängigen organischen Lösungsmitteln, verbesserte Filmbildungseigenschaften und eine deutlich verringerte Tendenz zur Kristallisation aus. Dadurch wird die Herstellung von Elektrolumineszenzvorrichtungen erleichtert und ihre Lebensdauer erhöht. Die Emissionseigenschaften der erfindungsgemäß eingesetzten Verbindungen können durch die Wahl geeigneter Substituenten über den ganzen Bereich des sichtbaren Spektrums eingestellt werden. Darüberhinaus erlaubt die kovalent gebundene Anordnung der zwei Teile der Spiroverbindung einen molekularen Aufbau in der Weise, daß bei beiden Hälften des Moleküls unabhängig bestimmte Eigenschaften eingestellt werden können. So kann die eine Hälfte z.B. Ladungstransport- oder Ladungsinjektionseigenschaften besitzen, während die andere lichtemittierende Eigenschaften besitzt. Die durch die kovalente Anknüpfung fixierte räumliche Nähe der beiden Hälften ist dabei günstig für die Energieübertragung (siehe z.B. B. Liphardt, W. Lüttke, Liebigs Ann. Chem. (1981)1118).

Besonders bevorzugt werden Spirobifluorenderivate gemäß den Ansprüchen 2 und 3 verwendet.

Gegenstand der Erfindung sind daher auch Spiroverbindungen der Formel (V), 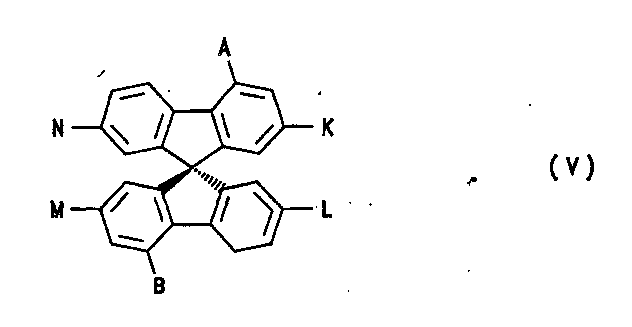 wie in Anspruch 4 definiert.

Bevorzugt sind Verbindungen der Formel (V) bei denen K, L, M, N und gegebenenfalls A, B aus der folgenden Gruppe G1 bis G14 ausgewählt sind: 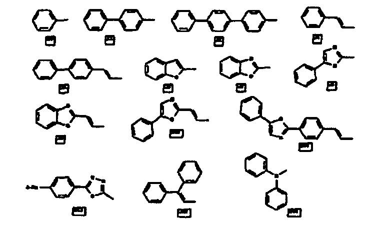 Insbesondere bevorzugte Spiroverbindungen der Formel (V) sind 2,2',4,4',7,7'- Hexakis(biphenylyl)-9,9'-spirobifluoren, 2,2',4,4',7,7'- Hexakis(terphenylyl)-9,9'-spirobifluoren, sowie die in den Tabellen 1 bis 4 aufgeführten Verbindungen, wobei die Abkürzungen G1 bis G14 die selben Bedeutungen wie in der Formel (V) haben.

**Tabelle 1:**

| Spiroverbindungen der Formel (V) A = B = G1 | | | | |
|---|---|---|---|---|
| Verbindung | K | L | M | N |
| Spiro-107 | G1 | G1 | G3 | G3 |
| Spiro-108 | G1 | G1 | G4 | G4 |
| Spiro-109 | G1 | G1 | G5 | G5 |
| Spiro-110 | G1 | G1 | G6 | G6 |
| Spiro-111 | G1 | G1 | G7 | G7 |
| Spiro-112 | G1 | G1 | G8 | G8 |
| Spiro-113 | G1 | G1 | G9 | G9 |
| Spiro-114 | G1 | G1 | G10 | G10 |
| Spiro-115 | G1 | G1 | G11 | G11 |
| Spiro-916 | G1 | G1 | G12 | G12 |
| Spiro-117 | G1 | G1 | G13 | G13 |
| Spiro-118 | G1 | G1 | G14 | G14 |
| Spiro-119 | G2 | G2 | G2 | G2 |
| Spiro-120 | G2 | G2 | G3 | G3 |
| Spiro-121 | G2 | G2 | G4 | G4 |
| Spiro-122 | G2 | G2 | G5 | G5 |
| Spiro-123 | G2 | G2 | G6 | G6 |
| Spiro-124 | G2 | G2 | G7 | G7 |
| Spiro-125 | G2 | G2 | G8 | G8 |
| Spiro-126 | G2 | G2 | G9 | G9 |
| Spiro-127 | G2 | G2 | G10 | G10 |
| Spiro-128 | G2 | G2 | G11 | G11 |
| Spiro-129 | G2 | G2 | G12 | G12 |
| Spiro-130 | G2 | G2 | G13 | G13 |
| Spiro-131 | G2 | G2 | G14 | G14 |
| Spiro-132 | G3 | G3 | G3 | G3 |
| Spiro-133 | G3 | G3 | G4 | G4 |
| Spiro-134 | G3 | G3 | G5 | G5 |
| Spiro-135 | G3 | G3 | G6 | G6 |
| Spiro-136 | G3 | G3 | G7 | G7 |
| Spiro-137 | G3 | G3 | G8 | G8 |
| Spiro-138 | G3 | G3 | G9 | G9 |
| Spiro-139 | G3 | G3 | G10 | G10 |
| Spiro-140 | G3 | G3 | G11 | G11 |
| Spiro-141 | G3 | G3 | G12 | G12 |
| Spiro-142 | G3 | G3 | G13 | G13 |
| Spiro-143 | G3 | G3 | G14 | G14 |
| Spiro-144 | G4 | G4 | G4 | G4 |
| Spiro-145 | G5 | G5 | G5 | G5 |
| Spiro-146 | G6 | G6 | G6 | G6 |
| Spiro-147 | G7 | G7 | G7 | G7 |
| Spiro-148 | G8 | G8 | G8 | G8 |
| Spiro-149 | G9 | G9 | G9 | G9 |
| Spiro-150 | G10 | G10 | G10 | G10 |
| Spiro-151 | G11 | G11 | G11 | G11 |
| Spiro-152 | G12 | G12 | G12 | G12 |
| Spiro-153 | G13 | G13 | G13 | G13 |
| Spiro-154 | G14 | G14 | G14 | G14 |
| Spiro-155 | H | H | G3 | G3 |
| Spiro-156 | H | H | G4 | G4 |
| Spiro-157 | H | H | G5 | G5 |
| Spiro-158 | H | H | G6 | G6 |
| Spiro-159 | H | H | G7 | G7 |
| Spiro-160 | H | H | G8 | G8 |
| Spiro-161 | H | H | G9 | G9 |
| Spiro-162 | H | H | G10 | G10 |
| Spiro-163 | H | H | G11 | G11 |
| Spiro-164 | H | H | G12 | G12 |
| Spiro-165 | H | H | G13 | G13 |
| Spiro-166 | H | H | G14 | G14 |
| Spiro-167 | G1 | G3 | G3 | G1 |
| Spiro-168 | G1 | G4 | G4 | G1 |
| Spiro-169 | G1 | G5 | G5 | G1 |
| Spiro-170 | G1 | G6 | G6 | G1 |
| Spiro-171 | G1 | G7 | G7 | G1 |
| Spiro-172 | G1 | G8 | G8 | G1 |
| Spiro-173 | G1 | G9 | G9 | G1 |
| Spiro-174 | G1 | G10 | G10 | G1 |
| Spiro-175 | G1 | G11 | G11 | G1 |
| Spiro-176 | G1 | G12 | G12 | G1 |
| Spiro-177 | G1 | G13 | G13 | G1 |
| Spiro-178 | G1 | G14 | G14 | G1 |
| Spiro-179 | G2 | G4 | G4 | G2 |
| Spiro-180 | G2 | G5 | G5 | G2 |
| Spiro-181 | G2 | G6 | G6 | G2 |
| Spiro-182 | G2 | G7 | G7 | G2 |
| Spiro-183 | G2 | G8 | G8 | G2 |
| Spiro-184 | G2 | G9 | G9 | G2 |
| Spiro-185 | G2 | G10 | G10 | G2 |
| Spiro-186 | G2 | G11 | G11 | G2 |
| Spiro-187 | G2 | G12 | G12 | G2 |
| Spiro-188 | G2 | G13 | G13 | G2 |
| Spiro-189 | G2 | G14 | G14 | G2 |
| Spiro-190 | G3 | G4 | G4 | G3 |
| Spiro-191 | G3 | G5 | G5 | G3 |
| Spiro-192 | G3 | G6 | G6 | G3 |
| Spiro-193 | G3 | G7 | G7 | G3 |
| Spiro-194 | G3 | G8 | G8 | G3 |
| Spiro-195 | G3 | G9 | G9 | G3 |
| Spiro-196 | G3 | G10 | G10 | G3 |
| Spiro-197 | G3 | G11 | G11 | G3 |
| Spiro-198 | G3 | G12 | G12 | G3 |
| Spiro-199 | G3 | G13 | G13 | G3 |
| Spiro-200 | G3 | G14 | G14 | G3 |
| Spiro-201 | H | G3 | G3 | H |
| Spiro-202 | H | G4 | G4 | H |
| Spiro-203 | H | G5 | G5 | H |
| Spiro-204 | H | G6 | G6 | H |
| Spiro-205 | H | G7 | G7 | H |
| Spiro-206 | H | G8 | G8 | H |
| Spiro-207 | H | G9 | G9 | H |
| Spiro-208 | H | G10 | G10 | H |
| Spiro-209 | H | G11 | G11 | H |
| Spiro-210 | H | G12 | G12 | H |
| Spiro-211 | H | G13 | G13 | H |
| Spiro-212 | H | G14 | G14 | H |

**Tabelle 2:**

| Spiroverbindungen der Formel (V) A = B = G2 | | | | |
|---|---|---|---|---|
| Verbindung | K | L | M | N |
| Spiro-213 | G1 | G1 | G3 | G3 |
| Spiro-214 | G1 | G1 | G4 | G4 |
| Spiro-215 | G1 | G1 | G5 | G5 |
| Spiro-216 | G1 | G1 | G6 | G6 |
| Spiro-217 | G1 | G1 | G7 | G7 |
| Spiro-218 | G1 | G1 | G8 | G8 |
| Spiro-219 | G1 | G1 | G9 | G9 |
| Spiro-220 | G1 | G1 | G10 | G10 |
| Spiro-221 | G1 | G1 | G11 | G11 |
| Spiro-222 | G1 | G1 | G12 | G12 |
| Spiro-223 | G1 | G1 | G13 | G13 |
| Spiro-224 | G1 | G1 | G14 | G14 |
| Spiro-225 | G2 | G2 | G2 | G2 |
| Spiro-226 | G2 | G2 | G3 | G3 |
| Spiro-227 | G2 | G2 | G4 | G4 |
| Spiro-228 | G2 | G2 | G5 | G5 |
| Spiro-229 | G2 | G2 | G6 | G6 |
| Spiro-230 | G2 | G2 | G7 | G7 |
| Spiro-231 | G2 | G2 | G8 | G8 |
| Spiro-232 | G2 | G2 | G9 | G9 |
| Spiro-233 | G2 | G2 | G10 | G10 |
| Spiro-234 | G2 | G2 | G11 | G11 |
| Spiro-235 | G2 | G2 | G12 | G12 |
| Spiro-236 | G2 | G2 | G13 | G13 |
| Spiro-237 | G2 | G2 | G14 | G14 |
| Spiro-238 | G3 | G3 | G3 | G3 |
| Spiro-239 | G3 | G3 | G4 | G4 |
| Spiro-240 | G3 | G3 | G5 | G5 |
| Spiro-241 | G3 | G3 | G6 | G6 |
| Spiro-242 | G3 | G3 | G7 | G7 |
| Spiro-243 | G3 | G3 | G8 | G8 |
| Spiro-244 | G3 | G3 | G9 | G9 |
| Spiro-245 | G3 | G3 | G10 | G10 |
| Spiro-246 | G3 | G3 | G11 | G11 |
| Spiro-247 | G3 | G3 | G12 | G12 |
| Spiro-248 | G3 | G3 | G13 | G13 |
| Spiro-249 | G3 | G3 | G14 | G14 |
| Spiro-250 | G4 | G4 | G4 | G4 |
| Spiro-251 | G5 | G5 | G5 | G5 |
| Spiro-252 | G6 | G6 | G6 | G6 |
| Spiro-253 | G7 | G7 | G7 | G7 |
| Spiro-254 | G8 | G8 | G8 | G8 |
| Spiro-255 | G9 | G9 | G9 | G9 |
| Spiro-256 | G10 | G10 | G10 | G10 |
| Spiro-257 | G11 | G11 | G11 | G11 |
| Spiro-258 | G12 | G12 | G12 | G12 |
| Spiro-259 | G13 | G13 | G13 | G13 |
| Spiro-260 | G14 | G14 | G14 | G14 |
| Spiro-261 | H | H | G3 | G3 |
| Spiro-262 | H | H | G4 | G4 |
| Spiro-263 | H | H | G5 | G5 |
| Spiro-264 | H | H | G6 | G6 |
| Spiro-265 | H | H | G7 | G7 |
| Spiro-266 | H | H | G8 | G8 |
| Spiro-267 | H | H | G9 | G9 |
| Spiro-268 | H | H | G10 | G10 |
| Spiro-269 | H | H | G11 | G11 |
| Spiro-270 | H | H | G12 | G12 |
| Spiro-271 | H | H | G13 | G13 |
| Spiro-272 | H | H | G14 | G14 |
| Spiro-273 | G1 | G3 | G3 | G1 |
| Spiro-274 | G1 | G4 | G4 | G1 |
| Spiro-275 | G1 | G5 | G5 | G1 |
| Spiro-276 | G1 | G6 | G6 | G1 |
| Spiro-277 | G1 | G7 | G7 | G1 |
| Spiro-278 | G1 | G8 | G8 | G1 |
| Spiro-279 | G1 | G9 | G9 | G1 |
| Spiro-280 | G1 | G10 | G10 | G1 |
| Spiro-281 | G1 | G11 | G11 | G1 |
| Spiro-282 | G1 | G12 | G12 | G1 |
| Spiro-283 | G1 | G13 | G13 | G1 |
| Spiro-284 | G1 | G14 | G14 | G1 |
| Spiro-285 | G2 | G4 | G4 | G2 |
| Spiro-286 | G2 | G5 | G5 | G2 |
| Spiro-287 | G2 | G6 | G6 | G2 |
| Spiro-288 | G2 | G7 | G7 | G2 |
| Spiro-289 | G2 | G8 | G8 | G2 |
| Spiro-290 | G2 | G9 | G9 | G2 |
| Spiro-291 | G2 | G10 | G10 | G2 |
| Spiro-292 | G2 | G11 | G11 | G2 |
| Spiro-293 | G2 | G12 | G12 | G2 |
| Spiro-294 | G2 | G13 | G13 | G2 |
| Spiro-295 | G2 | G14 | G14 | G2 |
| Spiro-296 | G3 | G4 | G4 | G3 |
| Spiro-297 | G3 | G5 | G5 | G3 |
| Spiro-298 | G3 | G6 | G6 | G3 |
| Spiro-299 | G3 | G7 | G7 | G3 |
| Spiro-300 | G3 | G8 | G8 | G3 |
| Spiro-301 | G3 | G9 | G9 | G3 |
| Spiro-302 | G3 | G10 | G10 | G3 |
| Spiro-303 | G3 | G11 | G11 | G3 |
| Spiro-304 | G3 | G12 | G12 | G3 |
| Spiro-305 | G3 | G13 | G13 | G3 |
| Spiro-306 | G3 | G14 | G14 | G3 |
| Spiro-307 | H | G3 | G3 | H |
| Spiro-308 | H | G4 | G4 | H |
| Spiro-309 | H | G5 | G5 | H |
| Spiro-310 | H | G6 | G6 | H |
| Spiro-311 | H | G7 | G7 | H |
| Spiro-312 | H | G8 | G8 | H |
| Spiro-313 | H | G9 | G9 | H |
| Spiro-314 | H | G10 | G10 | H |
| Spiro-315 | H | G11 | G11 | H |
| Spiro-316 | H | G12 | G12 | H |
| Spiro-317 | H | G13 | G13 | H |
| Spiro-318 | H | G14 | G14 | H |

**Tabelle 3:**

| Spiroverbindungen der Formel (V) A = B = G3 | | | | |
|---|---|---|---|---|
| Verbindung | K | L | M | N |
| Spiro-319 | G1 | G1 | G3 | G3 |
| Spiro-320 | G1 | G1 | G4 | G4 |
| Spiro-321 | G1 | G1 | G5 | G5 |
| Spiro-322 | G1 | G1 | G6 | G6 |
| Spiro-323 | G1 | G1 | G7 | G7 |
| Spiro-324 | G1 | G1 | G8 | G8 |
| Spiro-325 | G1 | G1 | G9 | G9 |
| Spiro-326 | G1 | G1 | G10 | G10 |
| Spiro-327 | G1 | G1 | G11 | G11 |
| Spiro-328 | G1 | G1 | G12 | G12 |
| Spiro-329 | G1 | G1 | G13 | G13 |
| Spiro-330 | G1 | G1 | G14 | G14 |
| Spiro-331 | G2 | G2 | G2 | G2 |
| Spiro-332 | G2 | G2 | G3 | G3 |
| Spiro-333 | G2 | G2 | G4 | G4 |
| Spiro-334 | G2 | G2 | G5 | G5 |
| Spiro-335 | G2 | G2 | G6 | G6 |
| Spiro-336 | G2 | G2 | G7 | G7 |
| Spiro-337 | G2 | G2 | G8 | G8 |
| Spiro-338 | G2 | G2 | G9 | G9 |
| Spiro-339 | G2 | G2 | G10 | G10 |
| Spiro-340 | G2 | G2 | G11 | G11 |
| Spiro-341 | G2 | G2 | G12 | G12 |
| Spiro-342 | G2 | G2 | G13 | G13 |
| Spiro-343 | G2 | G2 | G14 | G14 |
| Spiro-344 | G3 | G3 | G3 | G3 |
| Spiro-345 | G3 | G3 | G4 | G4 |
| Spiro-346 | G3 | G3 | G5 | G5 |
| Spiro-347 | G3 | G3 | G6 | G6 |
| Spiro-348 | G3 | G3 | G7 | G7 |
| Spiro-349 | G3 | G3 | G8 | G8 |
| Spiro-350 | G3 | G3 | G9 | G9 |
| Spiro-351 | G3 | G3 | G10 | G10 |
| Spiro-352 | G3 | G3 | G11 | G11 |
| Spiro-353 | G3 | G3 | G12 | G12 |
| Spiro-354 | G3 | G3 | G13 | G13 |
| Spiro-355 | G3 | G3 | G14 | G14 |
| Spiro-356 | G4 | G4 | G4 | G4 |
| Spiro-357 | G5 | G5 | G5 | G5 |
| Spiro-358 | G6 | G6 | G6 | G6 |
| Spiro-359 | G7 | G7 | G7 | G7 |
| Spiro-360 | G8 | G8 | G8 | G8 |
| Spiro-361 | G9 | G9 | G9 | G9 |
| Spiro-362 | G10 | G10 | G10 | G10 |
| Spiro-363 | G11 | G11 | G11 | G11 |
| Spiro-364 | G12 | G12 | G12 | G12 |
| Spiro-365 | G13 | G13 | G13 | G13 |
| Spiro-366 | G14 | G14 | G14 | G14 |
| Spiro-367 | H | H | G3 | G3 |
| Spiro-368 | H | H | G4 | G4 |
| Spiro-369 | H | H | G5 | G5 |
| Spiro-370 | H | H | G6 | G6 |
| Spiro-371 | H | H | G7 | G7 |
| Spiro-372 | H | H | G8 | G8 |
| Spiro-373 | H | H | G9 | G9 |
| Spiro-374 | H | H | G10 | G10 |
| Spiro-375 | H | H | G11 | G11 |
| Spiro-376 | H | H | G12 | G12 |
| Spiro-377 | H | H | G13 | G13 |
| Spiro-378 | H | H | G14 | G14 |
| Spiro-379 | G1 | G3 | G3 | G1 |
| Spiro-380 | G1 | G4 | G4 | G1 |
| Spiro-381 | G1 | G5 | G5 | G1 |
| Spiro-382 | G1 | G6 | G6 | G1 |
| Spiro-383 | G1 | G7 | G7 | G1 |
| Spiro-384 | G1 | G8 | G8 | G1 |
| Spiro-385 | G1 | G9 | G9 | G1 |
| Spiro-386 | G1 | G10 | G10 | G1 |
| Spiro-387 | G1 | G11 | G11 | G1 |
| Spiro-388 | G1 | G12 | G12 | G1 |
| Spiro-389 | G1 | G13 | G13 | G1 |
| Spiro-390 | G1 | G14 | G14 | G1 |
| Spiro-391 | G2 | G4 | G4 | G2 |
| Spiro-392 | G2 | G5 | G5 | G2 |
| Spiro-393 | G2 | G6 | G6 | G2 |
| Spiro-394 | G2 | G7 | G7 | G2 |
| Spiro-395 | G2 | G8 | G8 | G2 |
| Spiro-396 | G2 | G9 | G9 | G2 |
| Spiro-397 | G2 | G10 | G10 | G2 |
| Spiro-398 | G2 | G11 | G11 | G2 |
| Spiro-399 | G2 | G12 | G12 | G2 |
| Spiro-400 | G2 | G13 | G13 | G2 |
| Spiro-401 | G2 | G14 | G14 | G2 |
| Spiro-402 | G3 | G4 | G4 | G3 |
| Spiro-403 | G3 | G5 | G5 | G3 |
| Spiro-404 | G3 | G6 | G6 | G3 |
| Spiro-405 | G3 | G7 | G7 | G3 |
| Spiro-406 | G3 | G8 | G8 | G3 |
| Spiro-407 | G3 | G9 | G9 | G3 |
| Spiro-408 | G3 | G10 | G10 | G3 |
| Spiro-409 | G3 | G11 | G11 | G3 |
| Spiro-410 | G3 | G12 | G12 | G3 |
| Spiro-411 | G3 | G13 | G13 | G3 |
| Spiro-412 | G3 | G14 | G14 | G3 |
| Spiro-413 | H | G3 | G3 | H |
| Spiro-414 | H | G4 | G4 | H |
| Spiro-415 | H | G5 | G5 | H |
| Spiro-416 | H | G6 | G6 | H |
| Spiro-417 | H | G7 | G7 | H |
| Spiro-418 | H | G8 | G8 | H |
| Spiro-419 | H | G9 | G9 | H |
| Spiro-420 | H | G10 | G10 | H |
| Spiro-421 | H | G11 | G11 | H |
| Spiro-422 | H | G12 | G12 | H |
| Spiro-423 | H | G13 | G13 | H |
| Spiro-424 | H | G14 | G14 | H |

**Tabelle 4:**

| Spiroverbindungen der Formel (V) A = B = G12 | | | | |
|---|---|---|---|---|
| Verbindung | K | L | M | N |
| Spiro-425 | G1 | G1 | G3 | G3 |
| Spiro-426 | G1 | G1 | G4 | G4 |
| Spiro-427 | G1 | G1 | G5 | G5 |
| Spiro-428 | G1 | G1 | G6 | G6 |
| Spiro-429 | G1 | G1 | G7 | G7 |
| Spiro-430 | G1 | G1 | G8 | G8 |
| Spiro-431 | G1 | G1 | G9 | G9 |
| Spiro-432 | G1 | G1 | G10 | G10 |
| Spiro-433 | G1 | G1 | G11 | G11 |
| Spiro-434 | G1 | G1 | G12 | G12 |
| Spiro-435 | G1 | G1 | G13 | G13 |
| Spiro-436 | G1 | G1 | G14 | G14 |
| Spiro-437 | G2 | G2 | G2 | G2 |
| Spiro-438 | G2 | G2 | G3 | G3 |
| Spiro-439 | G2 | G2 | G4 | G4 |
| Spiro-440 | G2 | G2 | G5 | G5 |
| Spiro-441 | G2 | G2 | G6 | G6 |
| Spiro-442 | G2 | G2 | G7 | G7 |
| Spiro-443 | G2 | G2 | G8 | G8 |
| Spiro-444 | G2 | G2 | G9 | G9 |
| Spiro-445 | G2 | G2 | G10 | G10 |
| Spiro-446 | G2 | G2 | G11 | G11 |
| Spiro-447 | G2 | G2 | G12 | G12 |
| Spiro-448 | G2 | G2 | G13 | G13 |
| Spiro-449 | G2 | G2 | G14 | G14 |
| Spiro-450 | G3 | G3 | G3 | G3 |
| Spiro-451 | G3 | G3 | G4 | G4 |
| Spiro-452 | G3 | G3 | G5 | G5 |
| Spiro-453 | G3 | G3 | G6 | G6 |
| Spiro-454 | G3 | G3 | G7 | G7 |
| Spiro-455 | G3 | G3 | G8 | G8 |
| Spiro-456 | G3 | G3 | G9 | G9 |
| Spiro-457 | G3 | G3 | G10 | G10 |
| Spiro-458 | G3 | G3 | G11 | G11 |
| Spiro-459 | G3 | G3 | G12 | G12 |
| Spiro-460 | G3 | G3 | G13 | G13 |
| Spiro-461 | G3 | G3 | G14 | G14 |
| Spiro-462 | G4 | G4 | G4 | G4 |
| Spiro-463 | G5 | G5 | G5 | G5 |
| Spiro-464 | G6 | G6 | G6 | G6 |
| Spiro-465 | G7 | G7 | G7 | G7 |
| Spiro-466 | G8 | G8 | G8 | G8 |
| Spiro-467 | G9 | G9 | G9 | G9 |
| Spiro-468 | G10 | G10 | G10 | G10 |
| Spiro-469 | G11 | G11 | G11 | G11 |
| Spiro-470 | G12 | G12 | G12 | G12 |
| Spiro-471 | G13 | G13 | G13 | G13 |
| Spiro-472 | G14 | G14 | G14 | G14 |
| Spiro-473 | H | H | G3 | G3 |
| Spiro-474 | H | H | G4 | G4 |
| Spiro-475 | H | H | G5 | G5 |
| Spiro-476 | H | H | G6 | G6 |
| Spiro-477 | H | H | G7 | G7 |
| Spiro-478 | H | H | G8 | G8 |
| Spiro-479 | H | H | G9 | G9 |
| Spiro-480 | H | H | G10 | G10 |
| Spiro-481 | H | H | G11 | G11 |
| Spiro-482 | H | H | G12 | G12 |
| Spiro-483 | H | H | G13 | G13 |
| Spiro-484 | H | H | G14 | G14 |
| Spiro-485 | G1 | G3 | G3 | G1 |
| Spiro-486 | G1 | G4 | G4 | G1 |
| Spiro-487 | G1 | G5 | G5 | G1 |
| Spiro-488 | G1 | G6 | G6 | G1 |
| Spiro-489 | G1 | G7 | G7 | G1 |
| Spiro-490 | G1 | G8 | G8 | G1 |
| Spiro-491 | G1 | G9 | G9 | G1 |
| Spiro-492 | G1 | G10 | G10 | G1 |
| Spiro-493 | G1 | G11 | G11 | G1 |
| Spiro-494 | G1 | G12 | G12 | G1 |
| Spiro-495 | G1 | G13 | G13 | G1 |
| Spiro-496 | G1 | G14 | G14 | G1 |
| Spiro-497 | G2 | G4 | G4 | G2 |
| Spiro-498 | G2 | G5 | G5 | G2 |
| Spiro-499 | G2 | G6 | G6 | G2 |
| Spiro-500 | G2 | G7 | G7 | G2 |
| Spiro-501 | G2 | G8 | G8 | G2 |
| Spiro-502 | G2 | G9 | G9 | G2 |
| Spiro-503 | G2 | G10 | G10 | G2 |
| Spiro-504 | G2 | G11 | G11 | G2 |
| Spiro-505 | G2 | G12 | G12 | G2 |
| Spiro-506 | G2 | G13 | G13 | G2 |
| Spiro-507 | G2 | G14 | G14 | G2 |
| Spiro-508 | G3 | G4 | G4 | G3 |
| Spiro-509 | G3 | G5 | G5 | G3 |
| Spiro-510 | G3 | G6 | G6 | G3 |
| Spiro-511 | G3 | G7 | G7 | G3 |
| Spiro-512 | G3 | G8 | G8 | G3 |
| Spiro-513 | G3 | G9 | G9 | G3 |
| Spiro-514 | G3 | G10 | G10 | G3 |
| Spiro-515 | G3 | G11 | G11 | G3 |
| Spiro-516 | G3 | G12 | G12 | G3 |
| Spiro-517 | G3 | G13 | G13 | G3 |
| Spiro-518 | G3 | G14 | G14 | G3 |
| Spiro-519 | H | G3 | G3 | H |
| Spiro-520 | H | G4 | G4 | H |
| Spiro-521 | H | G5 | G5 | H |
| Spiro-522 | H | G6 | G6 | H |
| Spiro-523 | H | G7 | G7 | H |
| Spiro-524 | H | G8 | G8 | H |
| Spiro-525 | H | G9 | G9 | H |
| Spiro-526 | H | G10 | G10 | H |
| Spiro-527 | H | G11 | G11 | H |
| Spiro-528 | H | G12 | G12 | H |
| Spiro-529 | H | G13 | G13 | H |
| Spiro-530 | H | G14 | G14 | H |

Besonders bevorzugt sind Spiroverbindungen definiert in den Ansprüchen 5 und 6.

Die Herstellung der erfindungsgemäß verwendeten Spiroverbindungen erfolgt nach an sich literaturbekannten Methoden, wie sie in Standardwerken zur Organischen Synthese, z.B. Houben-Weyl, Methoden der Organischen Chemie, Georg-Thieme-Verlag, Stuttgart und in den entsprechenden Bänden der Serie "The Chemistry of Heterocyclic Compounds" von A. Weissberger und E. C. Taylor (Herausgeber) beschrieben werden.

Die Herstellung erfolgt dabei unter Reaktionsbedingungen, die für die genannten Umsetzungen bekannt und geeignet sind. Dabei kann auch von an sich bekannten, hier nicht näher erwähnten Varianten Gebrauch gemacht werden.

Verbindungen der Formel (III) werden beispielsweise ausgehend vom 9,9'-Spirobifluoren erhalten, dessen Synthese z.B. von R. G. Clarkson, M. Gomberg, J.Am.Chem.Soc. 52 (1930) 2881, beschrieben ist.

Die Herstellung von Verbindungen der Formal (IIIa) definiert in Anspruch 3 kann beispielsweise ausgehend von einer Tetrahalogenierung in den Positionen 2,2',7,7' des 9,9'-Spirobifluorens und anschließender Substitutionsreaktion erfolgen (siehe z.B. US 5,026,894) oder über eine Tetraacetylierung der Positionen 2,2',7,7' des 9,9'-Spirobifluorens mit anschließender C-C-Verknüpfung nach Umwandlung der Acetylgruppen in Aldehydgruppen oder Heterocyclenaufbau nach Umwandlung der Acetylgruppen in Carbonsäuregruppen erfolgen.

Die Herstellung von Verbindungen der Formel (IIIb) definiert in Anspruch 3 kann beispielsweise analog zu denen der Formel IIIa erfolgen, wobei die stöchiometrischen Verhältnisse bei der Umsetzung so gewählt werden, daß die Positionen 2,2' bzw. 7,7' funktionalisiert werden (siehe z.B. J. H. Weisburger, E. K. Weisburger, F. E. Ray, J. Am. Chem. Soc: 72 (1959) 4253; F. K. Sutcliffe, H. M. Shahidi, D. Paterson, J. Soc. Dyers Colour 94 (1978) 306 und G. Haas, V. Prelog, Helv. Chim. Acta 52 (1969) 1202).

Die Herstellung von Verbindungen der Formel (IIIc) definiert in Anspruch 3 kann beispielsweise über eine Dibromierung in 2,2'Stellung und anschließender Diacetylierung in 7,7' Stellung des 9,9'-Spirobifluorens und anschließende Umsetzung analog zu der der Verbindungen IIIa erfolgen.

Verbindungen der Formeln (IIIe)-(IIIg) definiert in Anspruch 3 sind beispielsweise durch Wahl geeignet substituierter Ausgangsverbindungen beim Aufbau des Spirobifluorens herstellbar, z.B. kann 2,7-Dibromspirobifluoren aus 2,7-Dibromfluorenon und 2,7-Dicarbethoxy-9,9-spirobifluoren durch Einsatz von 2,7-Dicarbethoxyfluorenon aufgebaut werden. Die freien 2,'7'-Positionen des Spirobifluorens können dann unabhängig weiter substituiert werden.

Für die Synthese der Gruppen K, L, M, N, sei beispielsweise verwiesen auf
DE-A 23 44 732, 24 50 088, 24 29 093, 25 02 904, 26 36 684, 27 01 591 und 27 52 975 für Verbindungen mit 1,4-Phenylen-Gruppen;
DE-A 26 41 724 für Verbindungen mit Pyrimidin-2,5-diyl-Gruppen;
DE-A 40 26 223 und EP-A 03 91 203 für Verbindungen mit Pyridin-2,5-diyl-Gruppen;
DE-A 32 31 462 für Verbindungen mit Pyridazin-3,6-diyl-Gruppen; N. Miyaura, T. Yanagi und A. Suzuki in Synthetic Communications 11 (1981) 513 bis 519, DE-A-3 930 663, M. J. Sharp, W. Cheng, V. Snieckus in Tetrahedron Letters 28 (1987), 5093; G. W. Gray in J. Chem. Soc. Perkin Trans II (1989) 2041 und Mol. Cryst. Liq. Cryst. 172 (1989) 165, Mol. Cryst. Liq. Cryst. 204 (1991) 43 und 91; EP-A 0 449 015; WO 89/12039; WO 89/03821; EP-A 0 354 434 für die direkte Verknüpfung von Aromaten und Heteroaromaten;

Die Herstellung disubstituierter Pyridine, disubstituierter Pyrazine, disubstituierter Pyrimidine und disubstituierter Pyridazine findet sich beispielsweise in den entsprechenden Bänden der Serie "The Chemistry of Heterocyclic Compounds" von A. Weissberger und E. C. Taylor (Herausgeber).

Erfindungsgemäß finden die beschriebenen Spiroverbindungen der Formeln (II) und (III) als Elektrolumineszenzmaterialien Verwendung, d.h., sie dienen als aktive Schicht in einer Elektrolumineszenzvorrichtung. Als aktive Schicht im Sinne der Erfindung gelten Elektrolumineszenzmaterialien, die befähigt sind bei Anlegen eines elektrischen Feldes Licht abzustrahlen (lichtemittierende Schicht), sowie Materialien, welche die Injektion und/oder den Transport der positiven und/oder negativen Ladungen verbessern (Ladungsinjektionsschichten und Ladungstransportschichten).

Erfindungsgemäße Elektrolumineszenzmaterialien zeichnen sich unter anderem auch durch eine außergewöhnliche Temperaturstabilität im Vergleich zu bekannten organischen Elektrolumineszenzmaterialien aus. Dies zeigt sich z.B. darin, daß das Emissionsmaximum der Verbindungen nach thermischer Belastung nur wenig, in anderen Fällen überhaupt nicht abnimmt und daß bei vielen Verbindungen sogar eine Zunahme des Emissionsmaximums nach thermischer Belastung festgestellt wird.

Gegenstand der Erfindung ist daher auch ein organisches Elektrolumineszenzmaterial, dadurch gekennzeichnet, daß sich sein Emissionsmaximum im Bereich von 400 bis 750 nm, gemessen bei Raumtemperatur, um nicht mehr als 15 %, relativ zum Ausgangszustand, vermindert, nachdem das Material, aufgetragen in einer Dicke von nicht mehr als 1 µm auf einem Quarzsubstrat, in einer inerten Atmosphäre bei einem Druck von nicht mehr als 1 mbar für 30 min auf 250°C erhitzt wurde.

Vorzugsweise beträgt die Verminderung des Emissionsmaximums nicht mehr als 10 %, besonders bevorzugt 5 %, relativ zum Ausgangszustand vor der thermischen Behandlung.

Ganz besonders bevorzugt sind Elektrolumineszenzmaterialien die keine Verminderung des Emissionsmaximums unter den oben angegebenen Bedingungen zeigen.

Insbesondere bevorzugt sind solche organische Elektrolumineszenzmaterialien, die unter den angegebenen Bedingungen eine Zunahme des Emissionsmaximums zeigen.

Unter inerter Atmosphäre wird vorzugsweise eine Stickstoff- oder Argonatmosphäre verstanden.

Gegenstand der Erfindung ist daher auch eine Elektrolumineszenzvorrichtung mit einer oder mehreren aktiven Schichten, die eine oder mehrere Verbindungen der Formel (II) und/oder (III) enthalten. Die aktive Schicht kann beispielsweise eine lichtemittierende Schicht und/oder eine Transportschicht und/oder ein Ladungsinjektionsschicht sein.

Der allgemeine Aufbau solcher Elektrolumineszenzvorrichtungen ist beispielsweise in US 4,539,507 und US 5,151,629 beschrieben.

Sie enthalten üblicherweise eine elektrolumineszierende Schicht zwischen einer Kathode und einer Anode, wobei mindestens eine der Elektroden transparent ist. Zusätzlich kann zwischen der elektrolumineszierenden Schicht und der Kathode eine Elektroneninjektions und/oder Elektronentransportschicht eingebracht sein undloder zwischen der elektrolumineszierenden Schicht und der Anode eine Lochinjektions und/oder Lochtransportschicht eingebracht sein. Als Kathode kann z.B. Ca, Mg, Al, In, Mg/Ag dienen. Als Anode können z.B. Au oder ITO (Indiumoxid/Zinnoxid auf einem transparentem Substrat, z.B. aus Glas oder einem transparenten Polymer) dienen.

Im Betrieb wird die Kathode auf negatives Potential gegenüber der Anode gesetzt, dabei werden Elektronen von der Kathode in die Elektroneninjektionsschicht /Elektronentransportschicht bzw. direkt in die lichtemittierende Schicht injiziert. Gleichzeitig werden Löcher von der Anode in die Lochinjektionsschicht/Lochtransportschicht bzw. direkt in die lichtemittierende Schicht injiziert.

Die injizierten Ladungsträger bewegen sich unter dem Einfluß der angelegten Spannung durch die aktiven Schichten aufeinander zu. Dies führt an der i Grenzfläche zwischen Ladungstransportschicht und lichtemittierender Schicht bzw. innerhalb der lichtemittierenden Schicht zu Elektronen/Loch-Paaren, die unter Aussendung von Licht rekombinieren.
Die Farbe des emittierten Lichtes kann durch die als lichtemittierende Schicht verwendete Verbindung variiert werden.

Elektrolumineszenzvorrichtungen finden Anwendung z.B. als selbstleuchtende Anzeigeelemente, wie Kontrollampen, alphanumerische Displays, Hinweisschilder, und in optoelektronischen Kopplem.

Die Erfindung wird durch die Beispiele näher erläutert, ohne sie darauf beschränken zu wollen.

### Beispiele

### A. Ausgangsverbindungen

### a) Synthese von 9,9'-Spirobifluoren

6,3 g Magnesiumspäne und 50 mg Anthracen werden in 120 ml trockenem Diethylether in einem 1 I Dreihalskoben mit Rückflußkühler unter Argon vorgelegt und das Magnesium 15 min mit Ultraschall aktiviert.
62 g 2-Brombiphenyl werden in 60 ml trockenem Diethylether gelöst. Etwa 10 ml dieser Lösung werden dem vorgelegten Magnesium zugegeben, um die Grignard-Reaktion zu starten.
Nach dem Anspringen der Reaktion wird unter weiterer Ultrabeschallung die 2-Brombiphenyl-Lösung so zugetropft, daß die Lösung gelinde am Rückfluß siedet. Nach beendeter Zugabe wird die Reaktionsmischung eine weitere Stunde am Rückfluß unter Ultraschall gekocht.
48,8 g 9-Fluorenon werden in 400 ml trockenem Diethylether gelöst und unter weiterer Ultrabeschallung der Grignard-Lösung zugetropft. Nach beendeter Zugabe wird weitere 2 h gekocht. Der nach Abkühlung der Reaktionsmischung ausgefallene gelbe Magnesiumkomplex des 9-(2-Biphenyl)-9-fluorenols wird abgesaugt und mit wenig Ether gewaschen. Der Magnesiumkomplex wird in 800 ml Eiswasser hydrolysiert, welches 40 g Ammoniumchlorid enthält. Nach 60 min Rühren wird das gebildete 9-(2-Biphenyl)-9-fluorenol abgesaugt, mit Wasser gewaschen und trocken gesaugt.
Das getrocknete 9-(2-Biphenyl)-9-fluorenol wird dann in 500 ml Eisessig in der Hitze gelöst. Zu dieser Lösung werden 0,5 ml konz. Salzsäure gegeben. Man läßt die Lösung einige Minuten kochen und fällt das gebildete 9,9'-Spirobifluoren aus der heißen Lösung mit Wasser (Wasserzugabe bis Trübung einsetzt). Nach Abkühlung wird das Produkt abgesaugt und mit Wasser gewaschen. Das getrocknete Produkt wird zur weiteren Reinigung aus Ethanol umkristallisiert. Man erhält 66 g (80 %, bez. auf 2-Brombiphenyl) 9,9'-Spirobifluoren als farblose Kristalle Smp. 198°C.

### b) 2,2'-Dibrom-9,9'-spirobifluoren

### (F. K. Sutcliffe, H. M. Shahidi, D. Patterson, J. Soc. Dyers & Colourists 94 (1978) 306)

3,26 g (10,3 mmol) 9,9'-Spirobifluoren werden in 30 ml Methylenchlorid gelöst und mit 5 mg FeCl₃ (wasserfrei) als Katalysator versetzt. Der Reaktionskolben wird vor Lichtzutritt geschützt. 1,12 ml (21,8 mmol) Brom in 5 ml Methylenchlorid werden innerhalb von 30 min unter Rühren zugetropft. Nach 24 h wird die resultierende braune Lösung mit gesättigter wäßriger NaHCO₃-Lösung und Wasser gewaschen, um überschüssiges Brom zu entfernen. Die organische Phase wird nach dem Trocknen über Na₂SO₄ am Rotationsverdampfer eingeengt. Der weiße Rückstand wird aus Methanol umkristallisiert, man erhält 3,45 g (70 %) der Dibromverbindung als farblose Kristalle, Smp. 240°C.

### c) 2,2',7,7'-Tetrabrom-9,9'-spirobifluoren

Zu einer Lösung von 3,16 g (10,0 mmol) 9,9'-Spirobifluoren in 30 ml Methylenchlorid werden 80 mg (0,5 mmol) wasserfreies FeCl₃ gegeben und 2,1 ml (41 mmol) Brom in 5 ml Methylenchlorid über 10 min hinweg zugetropft. Die Lösung wird 6 h am Rückfluß gekocht. Beim Abkühlen fällt das Produkt aus. Der Niederschlag wird abgesaugt und mit wenig kaltem Methylenchlorid gewaschen. Nach dem Trocknen erhält man 6,0 g (95 %) der Tetrabromverbindung als weißen Feststoff.

### d) 2-Bromo-9,9'-spirobifluoren und 2,2',7-Tribrom-9,9'-spirobifluoren sind bei veränderter Stöchiometrie auf analoge Weise herstellbar.

### e) 9,9'-Spirobifluoren-2,2'-dicarbonsäure

### aus 2,2'-Dibrom-9,9'-spirobifluoren über 2,2'-Dicyano-9,9'-spirobifluoren

1,19 g 2,2'-Dibromo-9,9'-spirobifluoren und 0,54 g CuCN werden in 5 ml DMF 6 h zum Rückfluß erhitzt. Die erhaltene braune Mischung wird in eine Mischung aus 3 g FeCl₃ (hydrat.) und 1,5 ml konz. Salzsäure in 20 ml Wasser gegossen. Die Mischung wird 30 min bei 60 bis 70EC gehalten, um den Cu-Komplex zu zerstören. Die heiße wäßrige Lösung wird zweimal mit Toluol extrahiert. Die organischen Phasen werden dann mit verdünnter Salzsäure, Wasser und 10 %iger wäßriger NaOH gewaschen. Die organische Phase wird filtriert und eingeengt. Der erhaltene gelbe Rückstand wird aus Methanol umkristallisiert. Man erhält 0,72 g (80 %) 2,2'-Dicyano-9,9'spirobifluoren als schwach gelbliche Kristalle (Schmelzbereich 215 bis 245°C).

3 g 2,2'-Dicyano-9,9'-spirobifluoren werden mit 25 ml 30 %iger wäßriger NaOH und 30 ml Ethanol für 6 h unter Rückfluß erhitzt. Das Dinatriumsalz der Spirobifluorendicarbonsäure fällt als gelber Niederschlag aus, der abfiltriert und in 25 %iger wäßriger HCI erhitzt wird, um die freie Säure zu gewinnen. Die Spirobifluorendicarbonsäure wird aus Eisessig umkristallisiert. Man erhält 2,2 g (66,6 %) weiße Kristalle (Smp. 376°C, IR-Bande 1685 cm⁻¹ C=O).

9,9'-Spirobifluoren-2,2',7,7'-tetracarbonsäure ist aus 2,2',7,7'-Tetrabrom-9,9'spirobifluoren in analoger Weise darstellbar.

### f) 9,9'-Spirobifluoren-2,2'-dicarbonsäure

aus 9,9'-Spirobifluoren über 2,2'-Diacetyl-9,9'-spirobifluoren (G. Haas, V. Prelog, Helv. Chim. Acta 52 (1969) 1202; V. Prelog, D. Bedekovic, Helv. Chim. Acta 62 (1979) 2285)

Eine Lösung von 3,17 g 9,9'-Spirobifluoren in 30 ml abs. Schwefelkohlenstoff wird nach Zugabe von 9,0 g feingepulvertem, wasserfreiem AlCl₃ während 10 min tropfenweise unter Rühren mit 1,58 g Acetylchlorid in 5 ml abs. Schwefelkohlenstoff versetzt und 1 Stunde unter Rückfluß gekocht. Die unter vermindertem Druck zur Trockene eingedampfte Mischung wird bei 0°C mit 100 g Eis und 50 ml 2n Salzsäure versetzt. Nach üblicher Aufarbeitung wird das Rohprodukt chromatographisch mit Benzol/Essigester (10:1) an Kieselgel getrennt. Man erhält 3,62 g (89 %) 2,2'-Diacetyl-9,9'-spirobifluoren (umkristallisiert aus Chloroform/Essigester, Smp. 255 bis 257°C) und 204 mg 2-Acetyl-9,9'-spirobifluoren (umkrist. aus Chloroform/Benzol, Smp. 225°C). [Daneben kann bei der Chromatographie auch 2,2',7-Triacetyl-9,9'-spirobifluoren (Smp. 258 bis 260°C) und 2,2',7,7'-Tetraacetyl-9,9'-spirobifluoren (Smp. > 300°C) isoliert werden, umkristallisiert aus Essigester/Hexan].
2,2',7-Triacetyl- und 2,2',7,7'-Tetraacetyl-9,9'-spirobifluoren können bei veränderter Stöchiometrie als Hauptprodukt erhalten werden.
Zu einer Lösung von 6,0 g Natriumhydroxid in 30 ml Wasser werden bei 0EC unter Rühren zuerst 7,2 g Brom und dann eine Lösung von 3,0 g 2,2'-Diacetyl-9,9'-spirobifluoren in wenig Dioxan zugetropft. Nach weiterem 1 stündigem Rühren bei Raumtemperatur wird die klare gelbe Lösung mit 1 g Natriumhydrogensulfit, gelöst in 20 ml Wasser, versetzt. Nach Ansäuern mit konz. Salzsäure wird das ausgefallene farblose Produkt abfiltriert und mit wenig Wasser gewaschen. Umkristallisation aus Ethanol liefert 9,9'-Spirobifluoren-2,2'-dicarbonsäure als wasserklare Prismen (Smp 352°C).

9,9'-Spirobifluoren-2-carbonsäure, 9,9'-Spirobifluoren-2,2',7-tricarbonsäure und 9,9'-Spirobifluoren-2,2',7,7'-tetracarbonsäure sind in analoger Weise darstellbar.

### g) 2,2'-Bis(brommethyl)-9,9'-spirobifluoren

### aus 2,2'-Dicarboxy-9,9'-spirobifluoren über 9,9'-Spirobifluoren-2,2'-dimethanol (V. Prelog, D. Bedekovicc, Helv. Chim. Acta 62 (1979) 2285)

Bei Raumtemperatur wurden 10 g einer 70 gew.-%igen Lösung von Natriumdihydro-bis(2-methoxyethoxy)-aluminat (Fluka) in Benzol langsam zu einer Suspension von 2,0 g 2,2'-Dicarboxy-9,9'-spirobifluoren (freie Carbonsäure) in 20 ml Benzol zugetropft. Nach 2 h Kochen unter Rückfluß, wobei sich die Carbonsäure auflöst, wird das überschüssige Reduktionsmittel bei 10°C mit Wasser zersetzt, das Gemisch mit konz. Salzsäure angesäuert und mit Chloroform ausgeschüttelt.

Die mit Wasser gewaschene und über Magnesiumsulfat getrocknete organische Phase wird eingedampft und der Rückstand aus Benzol umkristallisiert. Man erhält 1,57 g 9,9'-Spirobifluoren-2,2'-dimethanol (Smp. 254 bis 255°C).
Zu einer Lösung von 13,5 g 9,9'-Spirobifluoren-2,2'-dimethanol in 400 ml Benzol werden 91,5 g einer 33 %igen wäßrigen Lösung von Bromwasserstoff in Eisessig zugetropft und das Gemisch 7 h unter Rückfluß gekocht. Danach wird mit 200 ml Wasser versetzt und die mit Wasser gewaschene und über Magnesiumsulfat getrocknete organische Phase eingedampft. Die Chromatograhie an Kieselgel mit Benzol liefert 11,7 g 2,2'-Bis(brommethyl)-9,9'-spirobifluoren als farblose Plättchen
(Smp. 175 bis 177°C).

h) Eine Lösung von 380 mg 9,9'-Spirobifluoren-2,2'-dimethanol in 15 ml Toluol wird mit 5 g Chrom(VI)oxid auf Graphit (Seloxcette, Alpha Inorganics) versetzt und 48 h unter Stickstoff am Rückfluß gekocht. Dann wird durch eine Glasfiltemutsche abgenutscht und das Filtrat eingedampft. Chromatographie an Kieselgel mit Chloroform und Kristallisation aus Methylenchlorid/Ether liefert 152 mg 9,9'-Spirobifluoren-2,2'-dicarbaldehyd (Smp. > 300°C) und 204 mg 2'-Hydroxymethyl-9,9'-spirobifluoren-2-carbaldehyd
(Smp. 262 bis 263°C).

### i) 2,2'-Diamino-9,9'-spirobifluoren

Eine Mischung aus 150 ml konz. wäßriger HNO₃ und 150 ml Eisessig werden zu einer kochenden Lösung von 15,1 g 9,9'-Spirobifluoren in 500 ml Eisessig über einen Zeitraum von 30 min zugetropft anschließend wird die Lösung 75 min weiter refluxiert. Nach Abkühlung und Stehenlassen der Lösung für 1 h wird das gleiche Volumen Wasser zugesetzt und damit das Produkt ausgefällt. Nach dem Absaugen erhält man 18,5 g gelbe Kristalle (Smp. 220 bis 224°C) von 2,2'-Dinitro-9,9'-Spirobifluoren. Umkristallisation aus 250 ml Eisessig ergibt 12,7 g hellgelbe Kristallnadeln (Smp. 245 bis 249°C, analytisch rein 249 bis 250°C).
Eine Mischung aus 4,0 ml Dinitro-spirobifluoren und 4,0 g Eisenpulver werden in 100 ml Ethanol unter Rückfluß erhitzt, während 15 ml konz. HCI über einen Zeitraum von 30 min zugetropft werden. Nach weiteren 30 min Rückflußkochen wird überschüssiges Eisen abfiltriert. Das grüne Filtrat wird in eine Lösung aus 400 ml Wasser, 15 ml konz. NH₄OH und 20 g Na,K-Tartrat gegeben. Das weiße Diamin wird von der dunkelgrünen Lösung des Eisenkomplexes abfiltriert. Das Diamin wird zur Reinigung in verdünnter HCI gelöst und bei Raumtemperatur mit Aktivkohle (Darco) gerührt und abfiltriert. Die filtrierte Lösung wird unter Rühren
(KPG-Rührer) tropfenweise mit NH₄OH neutralisiert und das ausgefallene Produkt abgesaugt. Man erhält 3,5 g weißes 2,2'-Diamino-9,9'-spirobifluoren, das aus Ethanol umkristallisiert werden kann
(Smp. 243°C).

### j) Synthese von 2,2',7,7'-Tetrabromo-9,9'-spirobifluoren durch Bromierung von festem 9,9'-Spirobifluoren mit Bromdampf.

In eine flache Porzellan-Abdampfschale werden 3.16 g (10 mmol) fein gepulvertes 9,9'-Spirobifluoren gegeben. Diese Schale wird in einen Exsikkator, auf den gelochten Zwischenboden gestellt. Auf dem Boden des Exsikkators befinden sich ein einer Kristallisierschale 15.6 g (4.8 ml, 96 mmol) Brom. Der Exsikkator wird verschlossen, der Belüftungshahn jedoch geöffnet, damit das gebildete HBr entweichen kann. Der Exsikkator wird über Nacht in den Abzug gestellt. Am nächsten Tag wird die Porzellanschale mit dem durch Brom orange gefärbten Produkt aus dem Exsikkator genommen, und im Abzug noch mindestens 4 h stehen gelassen, damit überschüssiges Brom und HBr entweichen kann.
Das Produkt wird in 150 ml Dichlormethan gelöst und mit je 50 ml Natriumsulfitlösung (gesättigt), Natriumhydrogenkarbonatlösung (gesättigt) und Wasser farblos gewaschen. Die Dichlormethanlösung wird über Natriumsulfat getrocknet und einrotiert. Zur Reinigung wird aus Dichormethan/Pentan 4:1 umkristallisiert. Ausbeute 5.7 g (92%) farblose Kristalle.
¹H-NMR (CDCl₃, ppm): 6.83 (d, J = 1,83 Hz, 4 H, H-1,1',8,8'); 7.54 (dd, J = 7.93, 1.83 Hz, 4 H, H-3,3',6,6'); 7.68 (d, J = 7.93 Hz, 4 H, H-4,4',5,5').

### k) Synthese von 2,2',4,4',7,7'-Hexabromo-9,9'-spirobifluoren

Zu einer Lösung von 3.16 g (10 mmol) 9,9'-Spirobifluoren in 20 ml Methylenchlorid werden 200 mg wasserfreies FeCl₃ gegeben und mit Ultraschall behandelt. Der Reaktionskolben wird mit Al-Folie vor Lichtzutritt geschützt. Anschließend werden in der Siedehitze 9.85 g (3.15 ml, 62 mmol) Brom in 5 ml Methylenchlorid innerhalb von 15 min zugetropft. Die Lösung wird weitere 20 h am Rückfluß gekocht und mit Ultraschall behandelt. Nach Abkühlung wird Petrolether versetzt und abgesaugt. Zur weiteren Reinigung wird aus THF / Methanol umkristallisiert und 5 h bei 80°C getrocknet. Ausbeute 6.15 g (77 %) farblose Kristalle.
¹H-NMR (CDCl₃, ppm): 6.76 (d, J = 1,53 Hz, 2 H, H-1,1'); 6.84 (d, J = 1.83 Hz, 2 H, H-8,8'); 7.60 (dd, J = 8.54, 1.83 Hz, 2 H, H-6,6'); 7.75 (d, J = 1.53 Hz, 2 H, H-3,3'); 8.49 (d, J = 8.54 Hz, 2 H, H-5,5').

### I) Synthese von 2,7-Dibromo-9,9'-spirobifluoren

Das Grignardreagenz bereitet aus 0.72 g (30 mmol) Magnesiumspänen und 5.1 ml (30 mmol) 2-Brombiphenyl in 15 ml Diethylether wird im Verlauf von 2 h, unter Rühren (im Ultraschallbad) zu einer siedenden Suspension von 10.0 g (29.6 mmol) 2,7-Dibrom-9-fluorenon in 100 ml trockenem Diethylether getropft. Nach beendeter Zugabe wird 3 Stunden weiter gekocht. Nach Abkühlung über Nacht wird der ausgefallene Niederschlag abgesaugt und mit kaltem Ether gewaschen. Der abgesaugte Magnesiumkomplex wird in einer Lösung von 15 g Ammoniumchlorid in 250 ml Eiswasser hydrolisiert. Nach 1 h wird das gebildete 9-(2-Biphenylyl)-2,7-dibromo-9-fluorenol abgesaugt, mit Wasser gewaschen und trockengesaugt. Das getrocknete Fluorenol wird für die Ringschlußreaktion in 100 ml Eisessig, nach Zugabe von 3 Tropfen HCl conc. 6 Stunden gekocht. Man lässt über Nacht kristallisieren, saugt das gebildete Produkt ab und wäscht mit Eisessig und Wasser. Ausbeute: 11 g (77 %) 2,7-Dibromo-9,9'-spirobifluoren. Zur weiteren Reinigung kann aus THF umkristallisiert werden.
¹H-NMR (CDCl₃, ppm): 6.73 (d, J= 7.63 Hz, 2 H, H-1',8'); 6.84 (d, J = 1.83 Hz, 2 H, H-1,8); 7.15 (td, J = 7.63, 1.22 Hz., 2 H, H-2',7'); 7.41 (td, J = 7.63, 1.22 Hz, 2 H, H-3',6'); 7.48 (dd, J = 8.24, 1.83 Hz, 2 H, H-3,6); 7.67 (d, J = 8.24; 2 H; H-4,5); 7.85 (d, J = 7.63, 2 H, H-4',5').

### m) Synthese von 2,7-Dicarbethoxy-9,9'-spirobifluoren

Das Grignardreagenz bereitet aus 0.97 g (40 mmol) Magnesiumspänen und 9.32 g (6.8 ml, 40 mmol) 2-Brombiphenyl in 50 ml trockenem Diethylether wird im Verlauf von 2 h zu einer siedenden Lösung von 13 g (40 mmol) 2,7-Dicarbethoxy-9-fluorenon in 100 ml trockenem Diethylether getropft. Nach beendeter Zugabe wird 3 Stunden weiter gekocht. Nach Abkühlung über Nacht wird der ausgefallene Niederschlag abgesaugt und mit kaltem Ether gewaschen. Der abgesaugte Magnesiumkomplex wird in einer Lösung von 15 g Ammoniumchlorid in 250 ml Eiswasser hydrolisiert. Nach 1 h wird das gebildete 9-(2-Biphenylyl)-2,7-dicarbethoxy-9-fluorenol abgesaugt, mit Wasser gewaschen und trockengesaugt. Das getrocknete Fluorenol wird für die Ringschlußreaktion in 100 ml Eisessig, nach Zugabe von 3 Tropfen HCl conc. 6 Stunden gekocht. Man lässt über Nacht kristallisieren, saugt das gebildete Produkt ab und wäscht mit Eisessig und Wasser.
Ausbeute: 15.1 g (82 %) 2,7-Dicarbethoxy-9,9'-spirobifluoren. Zur weiteren Reinigung kann aus Ethanol umkristallisiert werden.
¹H-NMR (CDCl₃, ppm): 1.30 (t, J = 7.12 Hz, 6 H, Ester-CH₃); 4.27 (q, J = 7.12 Hz, 4 H, Ester-CH₂); 6.68 (d, J = 7.63 Hz, 2 H, H-1', 8'); 7.11 (td, J = 7.48, 1.22 Hz, 2H, H-2', 7'); 7.40 (td, J = 7.48, 1.22 Hz, 4 H, H-1, 8, 3', 6'); 7.89 (dt, J = 7.63, 0.92 Hz, 2 H, H-4', 5'); 7.94 (dd, J = 7.93, 0.6 Hz, 2 H, H-4, 5); 8.12 (dd, J = 7.93, 1.53 Hz, 2 H, H-3, 6).

### n) Synthese von 2,7-Dibromo,2',7'-dijodo-9,9'-spirobifluoren

In einem 250 ml Dreihalskolben mit Rückflußkühler und Tropftrichter wird bei 80°C eine Suspension von 2.37 g 2,7-Dibrom-9,9'-spirobifluoren in 50 ml Eisessig mit 5 ml Wasser versetzt und nach Zugabe von 2 ml konz. Schwefelsäure, 1.27 g Jod, 0.53 g Jodsäure sowie 5 ml Tetrachlorkohlenstoff bis zum Verschwinden der Jodfarbe gerührt. Anschließend wird abgesaugt und gut mit Wasser gewaschen. Nach dem Trocknen wird der Niederschlag in 150 ml Dichlormethan gelöst, und nacheinander mit Na₂SO₃-Lösung, NaHCO₃-Lösung und mit Wasser gewaschen. Die Dichlormethanphase wird über Na₂SO₄ getrocknet und anschließend eingeengt. Man erhält farblose Kristalle von 2,7-Dibromo,2',7'-dijodo-9,9'spirobifluoren in quantitativer Ausbeute. Zur weiteren Reinigung kann aus Dichlormethan/Pentan umkristallisiert werden.
¹H-NMR (CHCl₃, ppm):
6.80 (d, J = 1.83 Hz, 2 H), 6.99 (d, J = 1.53 Hz, 2 H), 7.51 (dd, J = 8.24, 1.83 Hz, 2 H), 7.54 (d, J = 7.93 Hz, 2 H), 7.65 (d, J = 8.24 Hz, 2 H), 7.72 (dd, J = 8.24, 1.53 Hz, 2 H).

### B. Synthesebeispiele

### Beispiel 1

### 2,2'-Bis(benzofuran-2-yl)-9,9'-spirobifluoren

### (analog zu W.Sahm, E.Schinzel, P.Jürges, Liebigs Ann.Chem. (1974) 523.)

2,7 g (22 mmol) Salicylaldehyd und 5,0 g (10 mmol) 2,2'-Bis(brommethyl)-9,9'spirobifluoren werden bei Raumtemperatur in 15 ml DMF gelöst und mit 0,9 g (22,5 mmol) pulverisiertem NaOH sowie einer Spatelspitze KJ versetzt. Man erhitzt zum Sieden und rührt 1 h bei Siedetemperatur. Nach Abkühlung versetzt man die Reaktionslösung mit einem Gemisch aus 0,5 ml konz. Salzsäure, 7 ml Wasser und 7 ml Methanol. Man rührt noch 1 h bei Raumtemperatur, saugt die kristallinen Reaktionsprodukte ab, wäscht zunächst mit kaltem Methanol, dann mit Wasser und trocknet im Vakuum bei 60EC. Man erhält 4,6 g (79 %) 2,2'-Bis(2-formylphenyloxymethyl)9,9'-spirobifluoren.
5,85 g (10 mmol) 2,2'-Bis(2-formylphenyloxymethyl)9,9'-spirobifluoren werden in 10 ml Toluol mit 2,1 g (22,5 mmol) frisch destilliertem Anilin versetzt. Man gibt eine Spatelspitze p-Toluolsulfonsäure zu und erhitzt am Wasserabscheider so lange zum Sieden, bis sich kein Wasser mehr abtrennt (ca. 3 bis 5 h). Beim Abkühlen des Reaktionansatzes fällt das korrespondierende bis-Benzylidenphenylamin kristallin aus. Es wird abgesaugt, mit Methanol gewaschen und im Vakuum bei 60EC getrocknet. Zur weiteren Reinigung kann aus DMF umkristallisiert werden. 7,35 g (10 mmol) des bis-Benzylidenphenylamins und 0,62 g (11 mmol) KOH werden unter Stickstoff in 30 ml DMF eingetragen. Anschließend erhitzt man unter Rühren 4 h auf 100°C. Nach Abkühlung auf Raumtemperatur wird der Niederschlag abgesaugt und mit wenig DMF und Wasser gewaschen. Nach Trocknen bei 60°C im Vakuumtrockenschrank kann das 2,2'-Bis(benzofuran-2-yl)-9,9'-spirobifluoren durch Umkristallisation aus Benzoesäuremethylester gereinigt werden.

### Beispiel 2

### 2,2',7,7'-Tetra(benzofuran-2-yl)-9,9'-spirobifluoren

kann bei entsprechend veränderter Stöchiometrie analog zu Beispiel 1 hergestellt werden.

### Beispiel 3

### 2,2',7,7'-Tetraphenyl-9,9'-spirobifluoren

5 g (7,9 mmol) 2,2',7,7'-Tetrabrom-9,9'-spirobifluoren, 3,86 g (31,6 mmol) Phenylboronsäure, 331,5 mg (1,264 mmol) Triphenylphosphin und 70,9 mg (0,316 mmol) Palladiumacetat werden in einer Mischung aus 65 ml Toluol und 40 ml wäßriger Natriumcarbonatlösung (2 M) aufgeschlämmt. Unter starkem Rühren wird die Mischung 24 h am Rückfluß gekocht. Nach Abkühlung auf Raumtemperatur wird abgesaugt, mit Wasser gewaschen und bei 50°C im Vakuum getrocknet. Man erhält 2,58 g. Das Filtrat wird mit 50 ml Toluol extrahiert und die getrocknete organische Phase zur Trockene eingeengt. Man erhält weitere 1,67 g.
Gesamtausbeute 4,25 g (86 %)

### Beispiel 4

### 2,2',7,7'-Tetrakis-(biphenyl)-9,9'-spirobifluoren

5 g (7,9 mmol) 2,2',7,7'-Tetrabromspirobifluoren, 6,57 g (33,2 mmol) Biphenylboronsäure, 331,5 mg (1,264 mmol) Triphenylphosphin und 70,9 mg (0,316 mmol) Palladiumacetat werden in einer Mischung aus 65 ml Toluol und 40 ml wäßriger Natriumcarbonatlösung (2 M) aufgeschlämmt. Unter starkem Rühren wird die Mischung 24 h am Rückfluß gekocht. Nach Abkühlung auf Raumtemperatur wird abgesaugt, mit Wasser gewaschen und bei 50°C im Vakuum getrocknet.
Ausbeute 5,95 g (81 %)

### Beispiel 5

### Synthese von 2,2',7,7'-Tetrabiphenylyl-9,9'-spirobifluoren

In einem 250 ml Zweihalskolben mit Rückflußkühler und KPG-Rührer werden 5,5 g Tetrabromspirobifluoren, 7.2 g Biphenylboronsäure und-400 mg Tetrakis(triphenylphosphin) palladium, in einer Mischung aus 100 ml Toluol und 50 ml Kaliumcarbonatlösung aufgeschlämmt. Unter Rühren mit einem KPG-Rührer und Schutzgasüberlagerung wird die Mischung 8 h am Rückfluß gekocht. Nach Abkühlung wird das Produkt abgesaugt, der Niederschlag mit Wasser gewaschen und getrocknet. Im Filtrat wird die Toluol-Phase abgetrennt und die wässrige Phase einmal mit Chloroform ausgeschüttelt. Die vereinigten organischen Phasen werden über Natriumsulfat getrocknet und einrotiert, man erhält so eine zweite Fraktion des Produktes. Die beiden Produktfraktionen werden vereinigt (8g) und in Chlorofrom gelöst. Die Chloroformlösung wird mit Aktivkohle aufgekocht und über eine kurze Säule mit Kieselgel filtriert. Nach dem Einrotieren und Umkristallisation aus Chloroform / Pentan erhält man farblose, unter UV-Beleuchtung blau fluoreszierende Kristalle. Schmelzpunkt 408°C (DSC).
¹H-NMR (CDCl₃, ppm): 7.14 (d, J = 1,53 Hz, 4 H ); 7.75 (dd, J = 7.93, 1.53 Hz, 4 H); 8.01 (d, J = 7.93 Hz, 4 H); 7.34 (dd, J = 7.32, 1.37 Hz, 4 H); 7.42 (t, J = 7.32 Hz, 8 H); 7.58 (24 H).

### Beispiel 6

### Synthese von 2,2',4,4',7,7'-Hexabiphenylyl-9,9'-spirobifluoren

In einem 250 ml Zweihalskolben mit Rückflußkühler, KPG-Rührer werden 1.6 g Hexabromspirobifluoren und 3 g Biphenylboronsäure in einer Mischung aus 50 ml Toluol und 50 ml 1 M Kaliumcarbonatlösung aufgeschlämmt. Die Mischung wird unter Stickstoff am Rückfluß gekocht und 115 mg Tetrakis(triphenylphosphin)palladium in 5 ml Toluol zugegeben. Die Mischung wird unter Rühren 7 h am Rückfluß gekocht. Nach Beendigung der Reaktion wird die abgekühlte Lösung abfiltriert und das Filtrat 2 x mit Wasser ausgeschüttelt (zur besseren Phasentrennung wird Chloroform zugesetzt. Die Organische Phase wird über Natriumsulfat getrocknet, über eine kurze Säule mit Kieselgel filtriert und anschließend einrotiert. Zur weiteren Reinigung wird aus Dichlormethan / Pentan umkristallisiert. Man erhält 2 g (80 %) farblose, unter UV-Beleuchtung blau fluoreszierende Kristalle.
¹³C-NMR [360 MHz.; ATP, breitbandentkoppelt] (CDCl₃, ppm):
65.94 (1C, Spiro-C); 126.95 (6C, CH), 126.97 (6C, CH), 127.17 (6C, CH), 127.35 (6C, CH), 127.36 (6C, CH), 127.39 (6C, CH), 127.52 (6C, CH), 128.73 (6C, CH), 128.75 (6C, CH), 128.94 (6C, CH), 129.90 (4 C, CH), 137.77 (2 C), 137.86 (2 C), 139.43 (2 C), 139.69 (2 C), 139.89 (2 C), 140.09 (2 C), 140.17 (2 C), 140.22 (2 C), 140.30 (2 C), 140.63 (2 C), 140.64 (2 C), 140.68 (2 C), 140.72 (2 C), 140.74 (2 C), 150.45 (2C), 150.92 (2C).

### Beispiel 7

### Synthese von 2,2'-Bis[(5(p-t-butylphenyl)-1,3,4-oxadiazol-2yl]-9,9'-spirobifluoren aus 9,9'-Spirobifluoren-2,2'-dicarbonsäurechlorid und 5(4-t-Butylphenyl)tetrazol

### a) Synthese von 5(4-t-Butylphenyl)tetrazol

In einem 250 ml Rundkolben mit Rückflußkühler werden 4.9 g *p*-*t*-Butylbenzonitril, 3.82 g Lithiumchlorid und 5.85 g Natriumazid und 8.2 g Triethylammoniumbromid in 100 ml DMF für 8 h auf 120°C erhitzt. Nach Abkühlung auf Raumtemperatur wird 100 ml Wasser zugesetzt und im Eisbad mit verd. Salzsäure versetzt, bis kein weiterer Niederschlag mehr fällt. Es wird abgesaugt, der Niederschlag mit Wasser gewaschen und getrocknet. Umkristallisation aus Ethanol / Wasser liefert 4.4 g farblose Kristalle.

### b) 9,9'-Spirobifluoren-2,2'-dicarbonsäurechlorid

In einem 100 ml Kolben mit Rückflußkühler und Trockenrohr werden 2 g (5 mmol) 9,9'-Spirobifluoren-2,2'-dicarbonsäure mit 20 ml (frisch destilliertem Thionylchlorid) und 3 Tropfen DMF, 4 h am Rückfluß gekocht. Nach Abkühlung wird der Rückflußkühler gegen eine Destillationsbrücke ausgetauscht und überschüssiges Thionylchlorid wird im Vakuum abdestilliert, dem Rückstand werden 40 ml Petrolether (30°-60°C) zugesetzt und abdestilliert, zurück bleibt das kristalline Säurechlorid.

### c) 2,2'-Bis[(5(p-t-butylphenyl)-1,3,4-oxadiazol-2yl]-9,9'-spirobifluoren

Dem Säurechlorid werden 2.0 g (11 mmol) 5(4-t-Butylphenyl)tetrazol gelöst in 20 ml wasserfreiem Pyridin zugesetzt und unter Schutzgas 2 h zum Rückfluß erhitzt. Nach Abkühlung wir die Mischung in 200 ml Wasser gegeben und 2 h stehen gelassen. Das ausgefallene Oxadiazolderivat wird abgesaugt, mit Wasser gewaschen und im Vakuum getrocknet. Anschließend wird über Kieselgel mit Chloroform/Essigester (99:1) chromatographiert und aus Chloroform / Pentan umkristallisiert. Man erhält 2.4 g farblose Kristalle.
¹H-NMR (CDCl₃), ppm):
1.31 (s, 18 H, t-Butyl), 6.77 (d, J = 7.32 Hz, 2 H), 7.18 (td, J = 7.48, 1.22 Hz, 2 H), 7.44 (td, J = 7.40, 1.22 Hz, 2 H), 7.46 (d, J = 8.54 Hz, 4 H), 7.50 (d, J = 1.22 Hz, 2 H), 7.94 (d, J = 8.54 Hz, 4 H), 8.02 (d, J = 7.93 Hz, 6 H), 8.20 (dd, J = 7.93, 1.53 Hz, 2 H).

### C. Anwendungsbeispiel

### 2,2',7,7'-Tetrakis-(biphenyl)-9,9'-spirobifluoren wird in Chloroform gelöst

(30 mg/ml) und mittels spin-coating (1000 upm) auf einen mit Indium/Zinn-Oxid (ITO) beschichteten Glasträger aufgebracht, wobei ein homogener, transparenter Film gebildet wird. Auf diesen Film wird durch Vakuumbedampfung eine Elektrode aus Mg/Ag (80/20) aufgebracht. Beim Anlegen einer elektrischen Spannung zwischen der ITO-Elektrode und der Metallelektrode, wobei die Metallelektrode negativ gegenüber der ITO-Elektrode gepolt ist, wird eine blaue Elektrolumineszenz beobachtet.

## Patentansprüche

1. Verwendung von Spiroverbindungen der allgemeinen Formel (II),
wobei die Benzogruppen unabhängig voneinander substituiert und/oder anelliert sein können 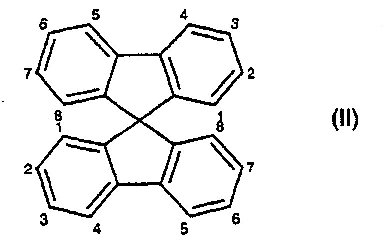 in Elektrolumineszenzvorrichtungen.

2. Verwendung nach Anspruch 1, **dadurch gekennzeichnet, daß** ein Spirobifluorenderivat der Formel (III) eingesetzt wird, 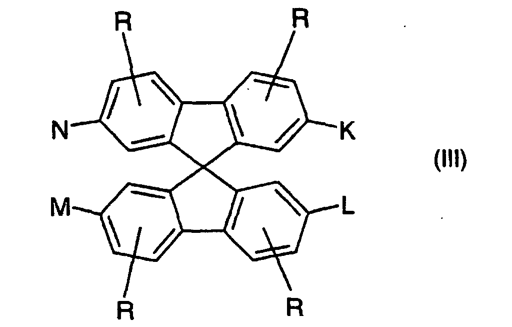 wobei die Symbole und Indizes folgende Bedeutungen haben:
K, L, M, N sind gleich oder verschieden 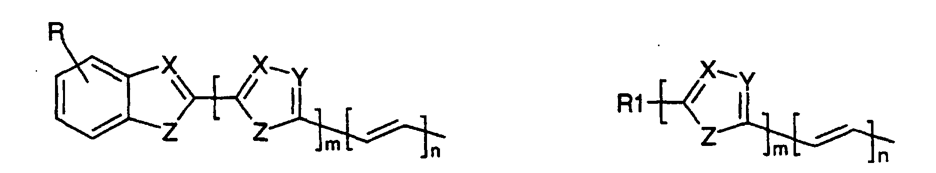 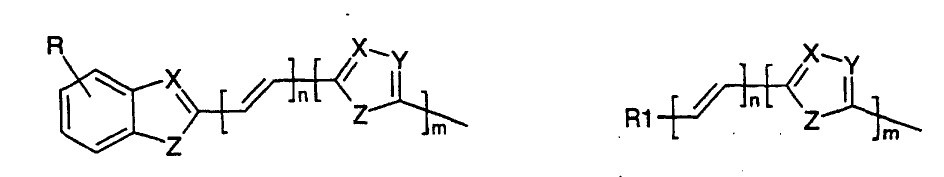 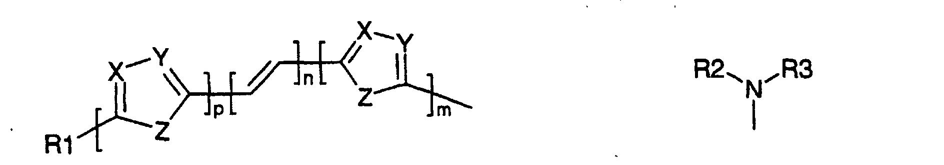 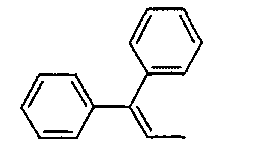
R kann, gleich oder verschieden, die gleichen Bedeutungen wie K, L, M, N haben oder ist -H, eine lineare oder verzweigte Alkyl, Alkoxy oder Estergruppe mit 1 bis 22 C-Atomen, -CN, -NO₂, -NR²R³, -Ar oder -O-Ar;
Ar ist Phenyl, Biphenyl, 1-Naphthyl, 2-Naphthyl, 2-Thienyl, 2-Furanyl, wobei jede dieser Gruppen einen oder zwei Reste R tragen kann,
m, n, p sind 0, 1, 2 oder 3;
X, Y sind gleich oder verschieden CR, N;
Z ist -O-, -S-, -NR¹ -, -CR¹ R⁴-, -CH=CH-, -CH=N-;
R¹, R⁴ können, gleich oder verschieden, die gleichen Bedeutungen wie R haben
R², R³ sind gleich oder verschieden H, eine lineare oder verzweigte Alkylgruppe mit 1 bis 22 C-Atomen, -Ar, 3-Methylphenyl.

3. Verwendung nach Anspruch 2, **dadurch gekennzeichnet, daß** ein Spirobifluorenderivat der Formel (IIIa) bis (IIIg) eingesetzt wird,
IIIa) K = L = M = N und ist aus der Gruppe: 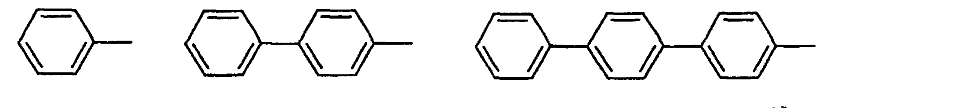 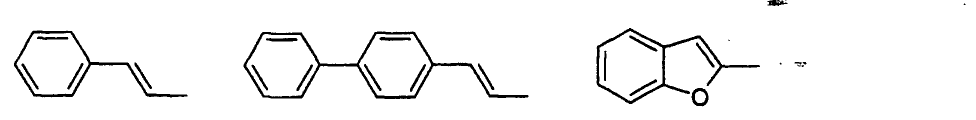 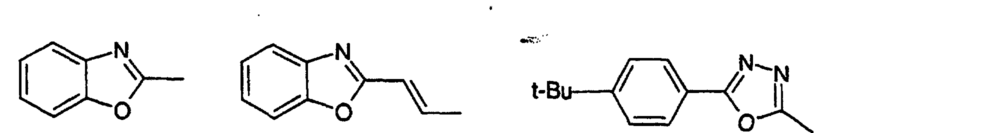 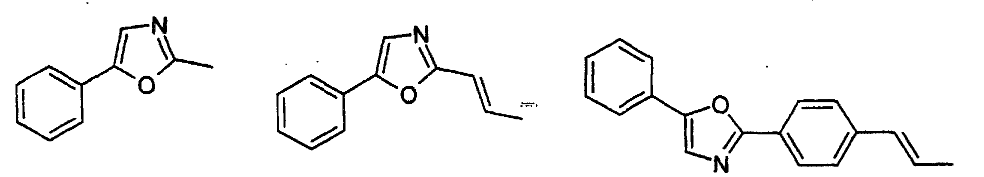 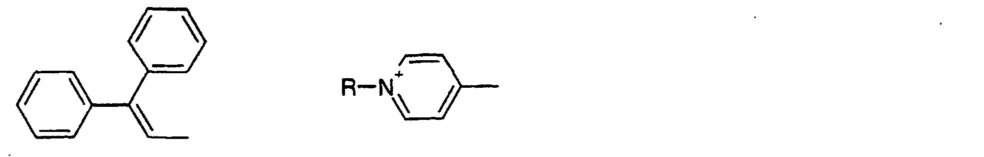 R = C₁-C₂₂-Alkyl, C₂H₄SO₃-
Illb) K = M = H und N = L und ist aus der Gruppe: 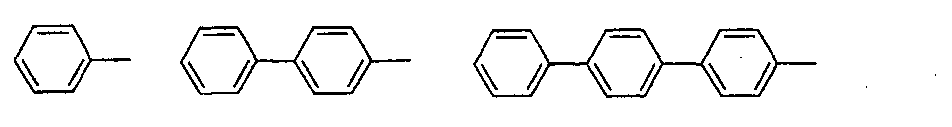 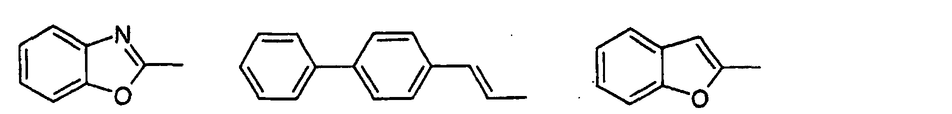 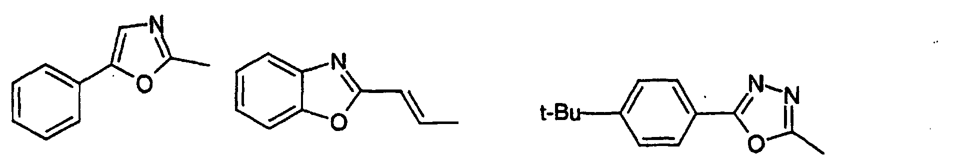 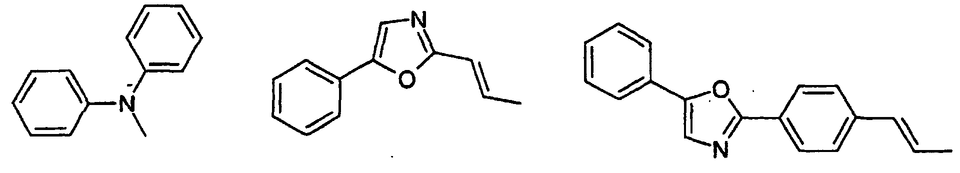
IIIc) K = M und ist aus der Gruppe: 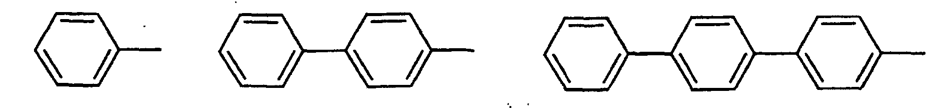 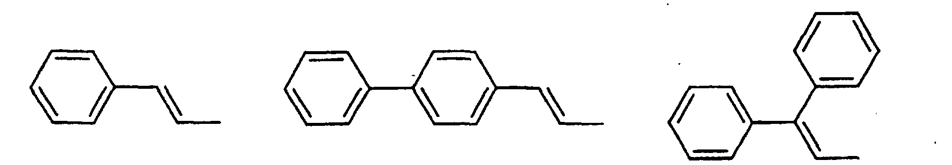 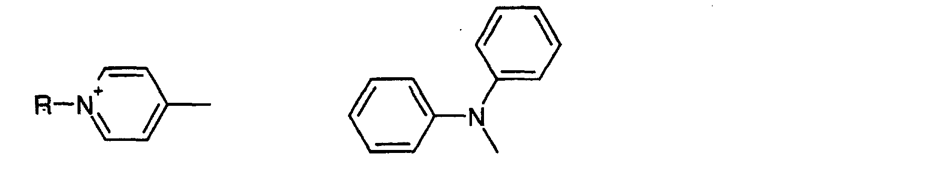 R = C₁-C₂₂-Alkyl, C₂H₄SO₃-
und N = L und ist aus der Gruppe: 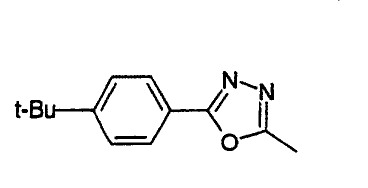 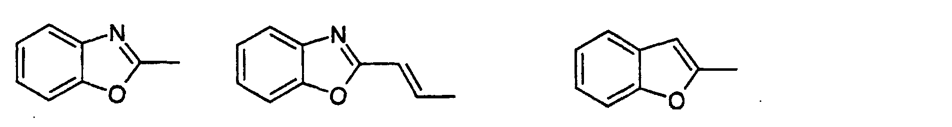 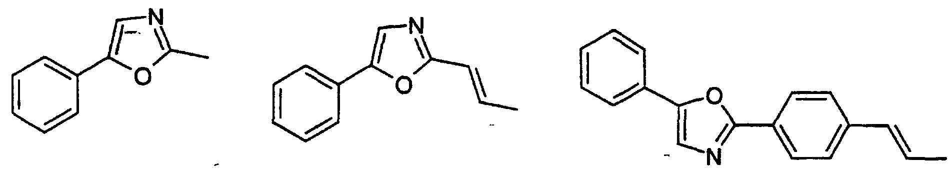
IIId) K = M und ist aus der Gruppe: 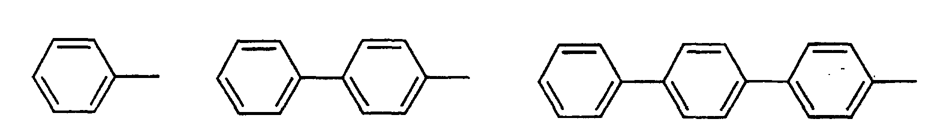 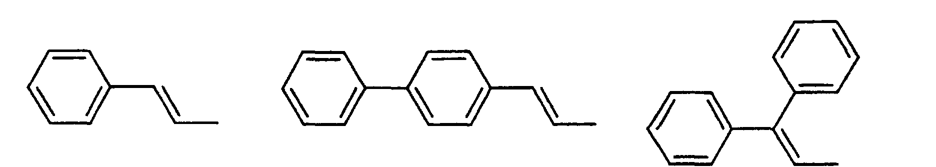 und N = L und ist aus der Gruppe: 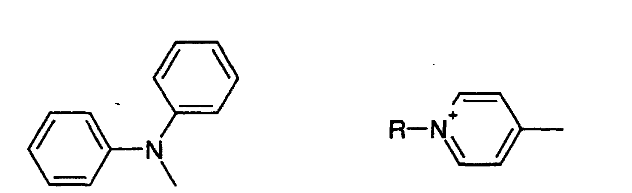 R = C₁-C₂₂-Alkyl, C₂H₄SO₃-
IIIe) K=L=H und M=N und ist aus der Gruppe: 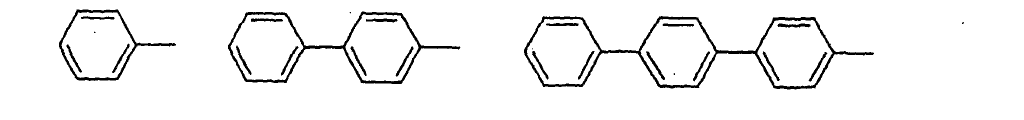 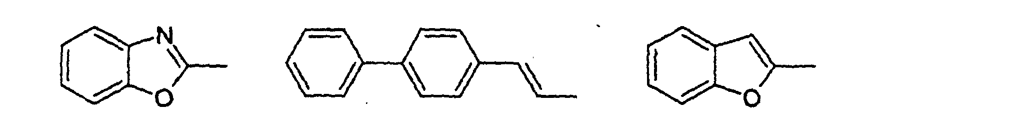 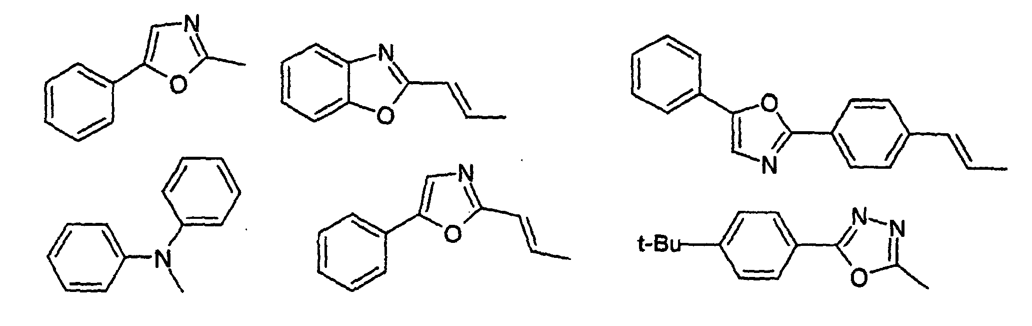
IIIf) K = L und ist aus der Gruppe: 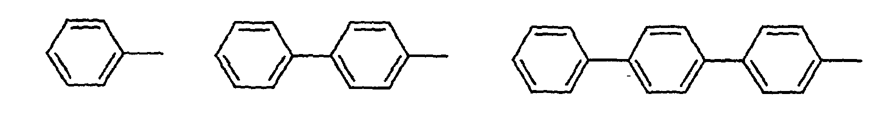 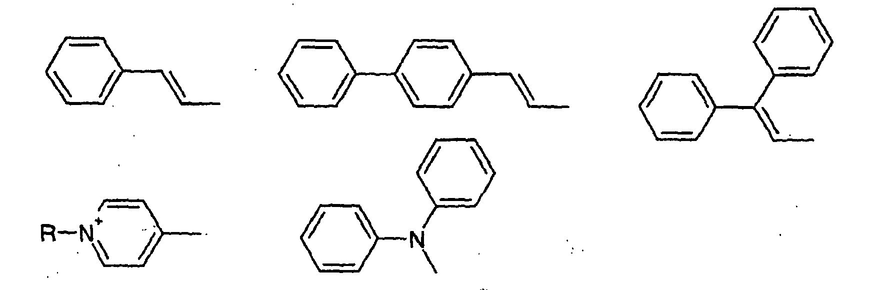 R = C₁-C₂₂-Alkyl, C₂H₄SO₃-
und M = N und ist aus der Gruppe: 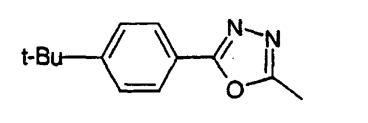 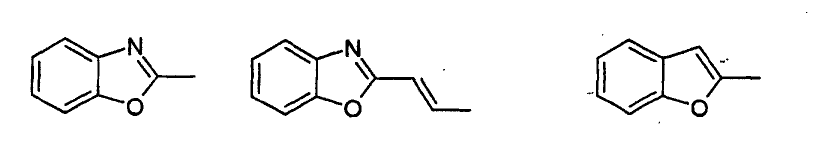 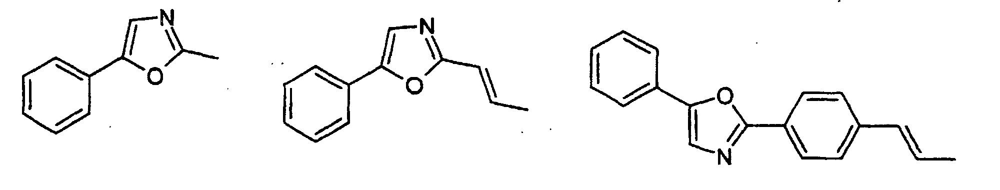
IIIg) K = L und ist aus der Gruppe: 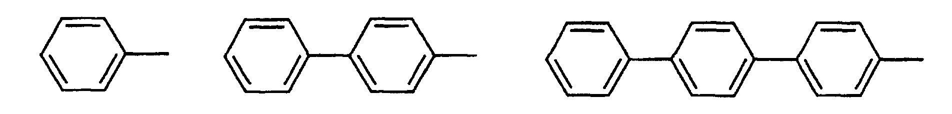 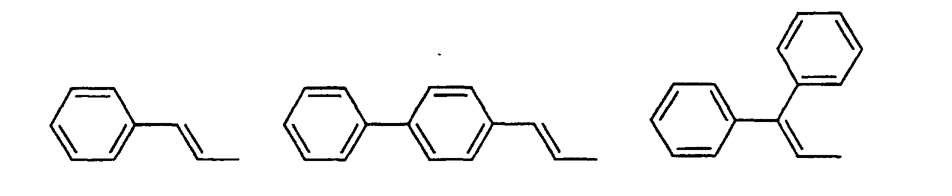 und M = N und ist aus der Gruppe: 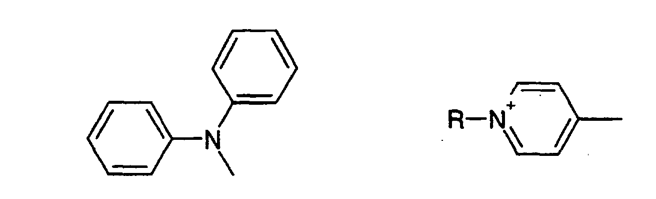 R = C₁-C₂₂-Alkyl, C₂H₄SO₃-

4. Spiroverbindung der Formel (V), 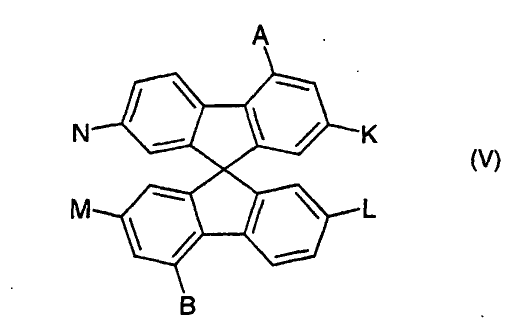 wobei die Symbole folgende Bedeutungen haben:
A, B sind gleich oder verschieden 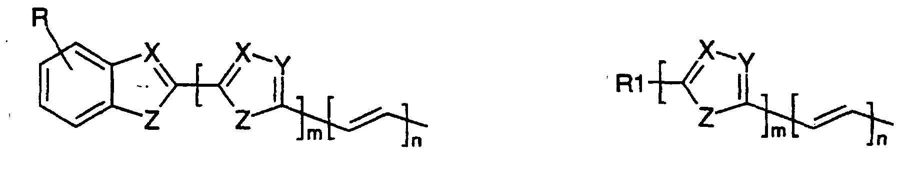 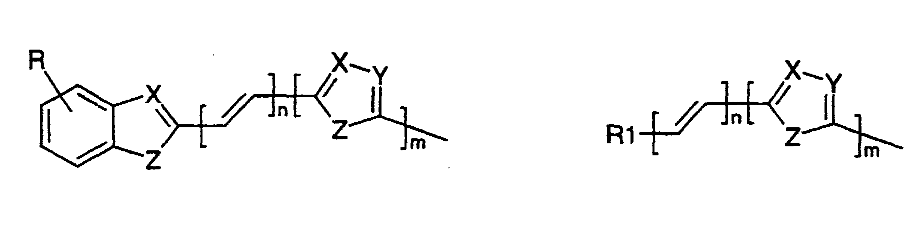 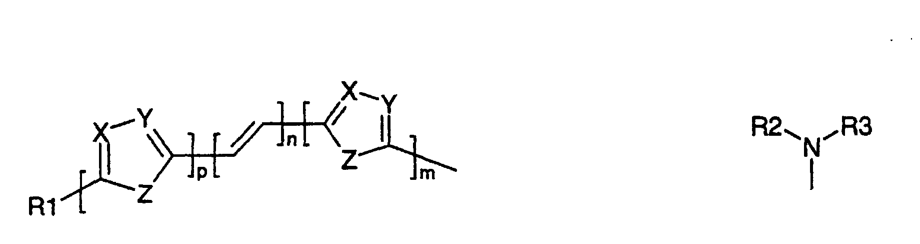
und
A, B können auch gleich oder verschieden eine lineare oder verzweigte Alkyl-, Alkyloxyoder Estergruppe mit 1 bis 22 C-Atomen, -CN, -NO₂, -Ar oder -O-Ar sein;
K, L, M, N sind gleich oder verschieden 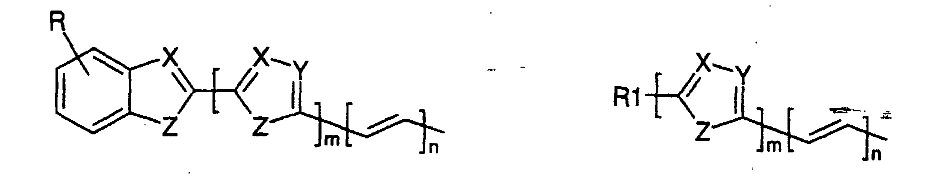 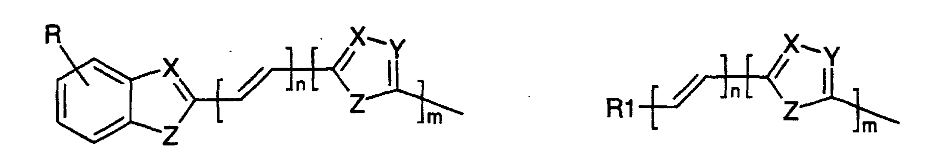 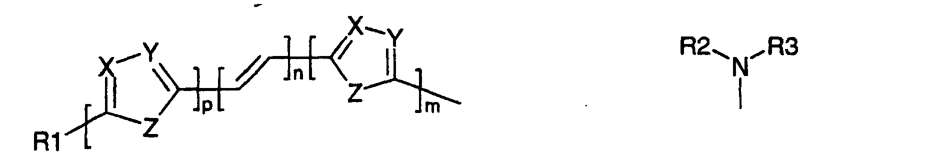 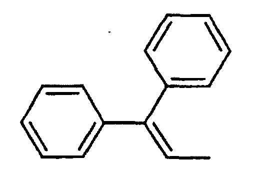
R ist,-H, eine lineare oder verzweigte Alkyl, Alkoxy oder Estergruppe mit 1 bis 22 C-Atomen, -CN, -NO₂, -NR²R³, -Ar oder -O-Ar;
Ar ist Phenyl, Biphenyl, 1-Naphthyl, 2-Naphthyl, 2-Thienyl, 2-Furanyl, wobei jede dieser Gruppen einen oder zwei Reste R tragen kann;
m, n, p sind 0,1,2 oder 3;
X, Y sind gleich oder verschieden CR, N;
Z ist -O-, -S-, -NR¹-, -CR¹R⁴-, -CH=CH-, -CH=N-;
R¹ ist eine lineare oder verzweigte Alkyl, Alkoxy oder Estergruppe mit 1 bis 22 C-Atomen, -CN, -NO₂, -NR²R³, -Ar oder -O-Ar;
R⁴ ist -H, eine lineare oder verzweigte Alkyl, Alkoxy oder Estergruppe mit 1 bis 22 C-Atomen, -CN, -NO₂, -NR²R³, -Ar oder -O-Ar;
R², R³ sind gleich oder verschieden eine lineare oder verzweigte Alkylgruppe mit 1 bis 22 C-Atomen, -Ar oder 3-Methylphenyl.

5. Spiroverbindung der Formel

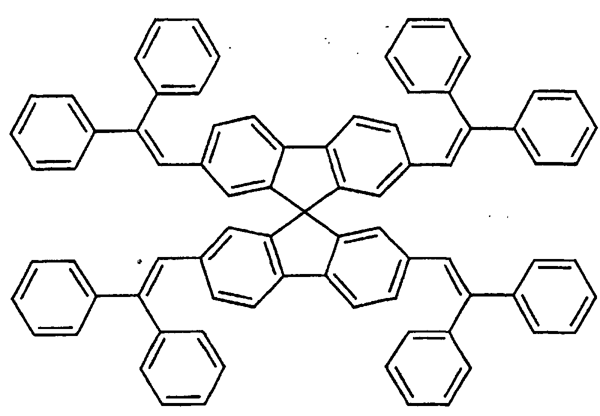

6. Spiroverbindung der Formel

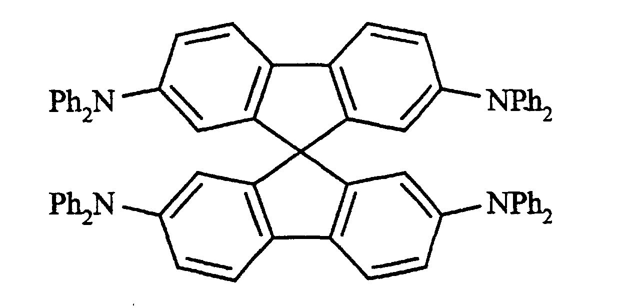

7. Verwendung nach einem oder mehreren der Ansprüche 1 bis 3, **dadurch gekennzeichnet, daß** die Spiroverbindung als lichtemittierende Schicht dient.

8. Verwendung nach einem oder mehreren der Ansprüche 1 bis 3, **dadurch gekennzeichnet, daß** die Spiroverbindung als Transportschicht dient.

9. Verwendung nach einem oder mehreren der Ansprüche 1 bis 3, **dadurch gekennzeichnet, daß** die Spiroverbindung zur Ladungsinjektion dient.

10. Elektrolumineszenzvorrichtung, enthaltend eine aktive Schicht, die eine oder mehrere Verbindungen der Formel (II) bis (III) nach einem oder mehreren der Ansprüche 1 bis 3 enthält.

11. Elektrolumineszenzvorrichtung nach Anspruch 10, **dadurch gekennzeichnet, daß** die aktive Schicht eine lichtemittierende Schicht ist.

12. Elektrolumineszenzvorrichtung nach Anspruch 10, **dadurch gekennzeichnet, daß** die aktive Schicht eine Transportschicht ist.

13. Elektrolumineszenzvorrichtung nach Anspruch 10, **dadurch gekennzeichnet, daß** die aktive Schicht eine Ladungsinjektionsschicht ist.

## Claims

1. Use of spiro compounds of the formula (II) where the benzo groups can be substituted and/or fused independently of one another. 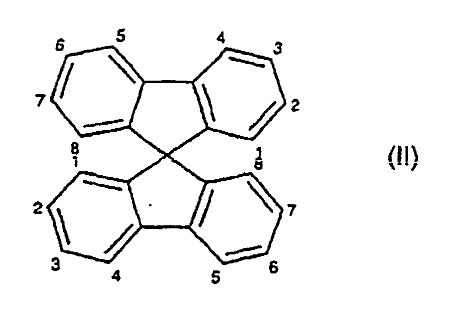 in electroluminescence devices.

2. Use as claimed in claim 1, wherein use is made of a spirobifluorene derivative of the formula (III) 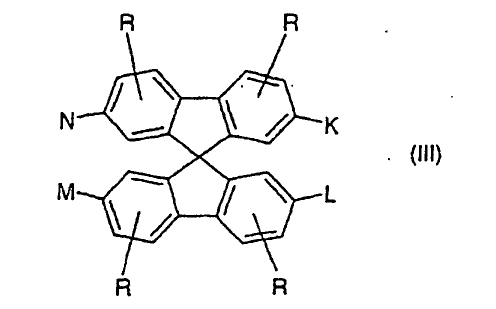 where the symbols and indices have the following meanings:
K, L, M, N are identical or different and are 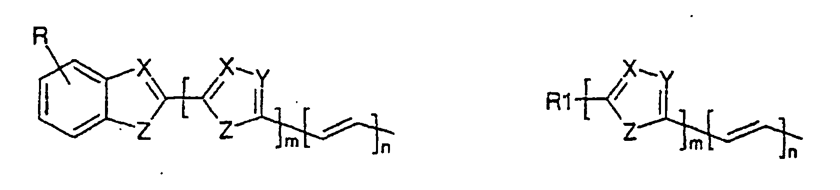 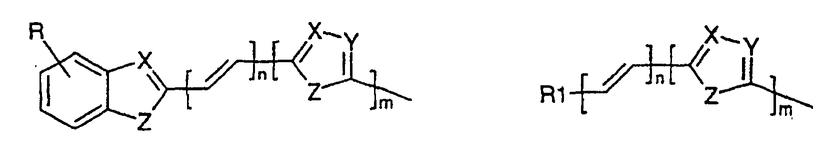 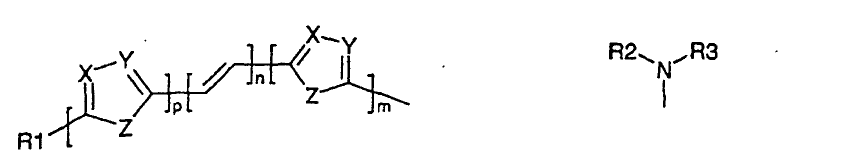 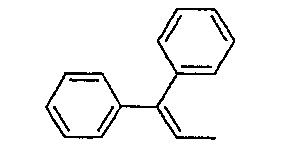
R can be identical or different on each appearance and have the same meanings as K, L, M, N or is H, a linear or branched alkyl, alkoxy or ester group having from 1 to 22 carbon atoms, -CN, -NO₂, -NR²R³, -Ar or -O-Ar;
Ar is phenyl, biphenyl, 1-naphthyl, 2-naphthyl, 2-thienyl, 2-furanyl, with each of these groups being able to bear one or two radicals R,
m, n, p are 0, 1, 2 or 3;
X, Y are identical or different and are CR or nitrogen;
Z is -O-, -S-, -NR¹-, -CR¹R⁴-, -CH=CH-, -CH=N-;
R¹, R⁴ can be identical or different and have the same meanings as R;
R², R³ are identical or different and are H, a linear or branched alkyl group having from 1 to 22 carbon atoms, -Ar, 3-methylphenyl.

3. Use as claimed in claim 2, wherein use is made of a spirobifluorene derivative of the formulae (IIIa) to (IIIg)
IIIa) K = L = M = N and is selected from the group consisting of: 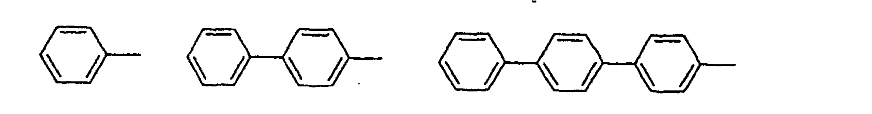 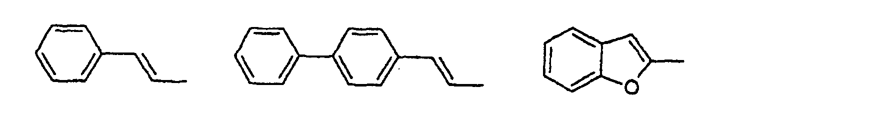 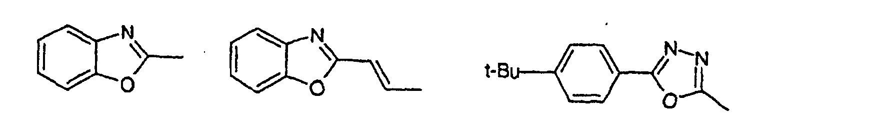 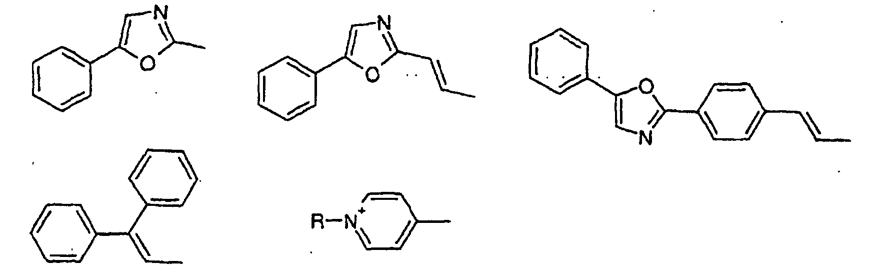 R = C₁-C₂₂-alkyl, C₂H₄SO₃-
IIIb) K = M = H and N = L and is selected from the group consisting of: 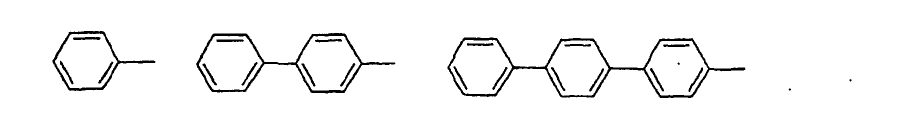 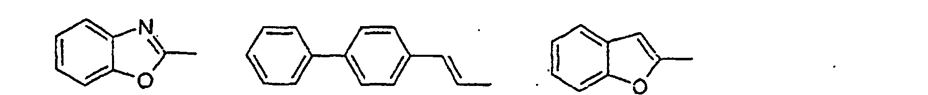 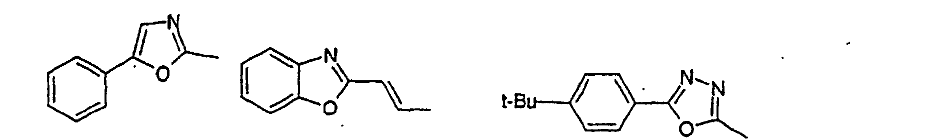 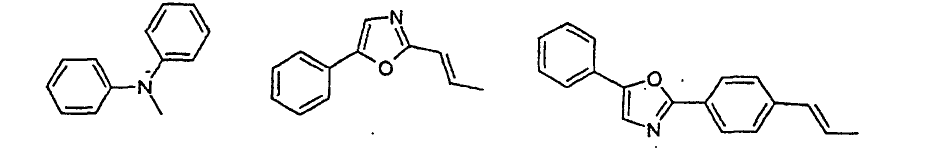
IIIc) K = M and is selected from the group consisting of: 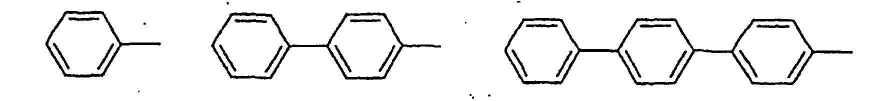 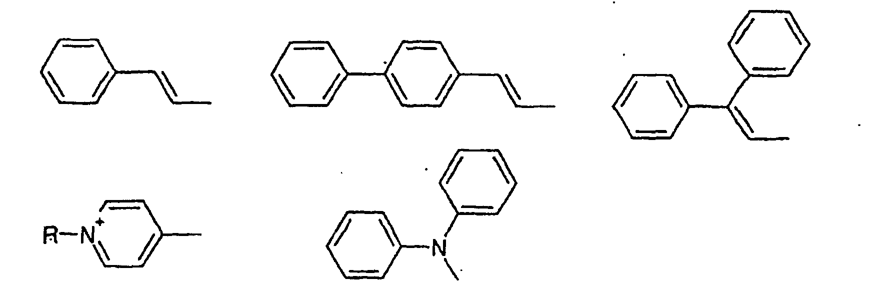 R = C₁-C₂₂-alkyl, C₂H₄SO₃-
and N = L and is selected from the group consisting of: 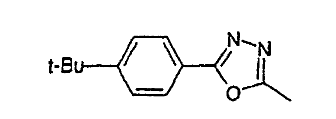 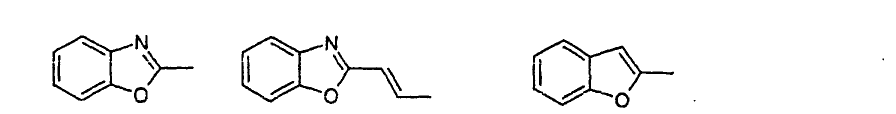 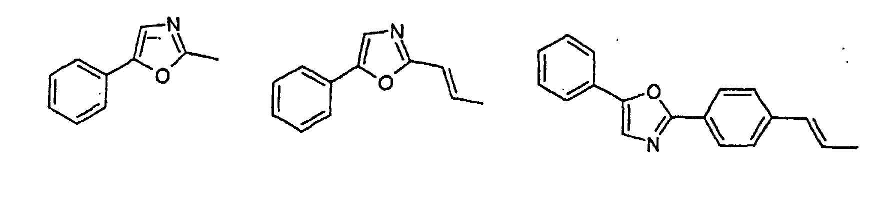
IIId) K = M and is selected from the group consisting of: 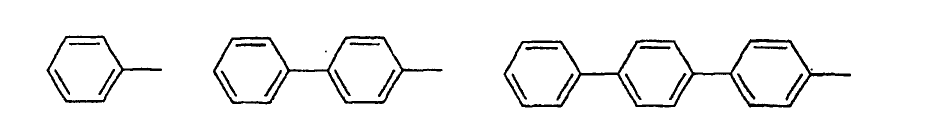 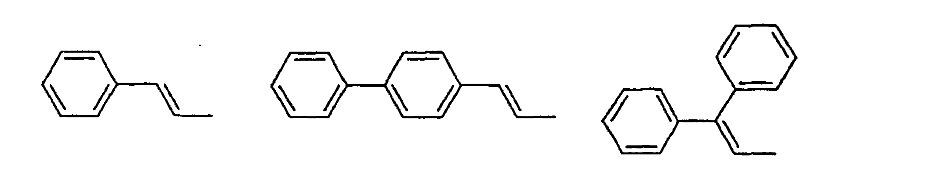 and N = L and is selected from the group consisting of: 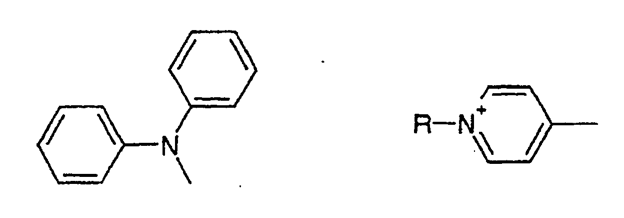 R = C₁-C₂₂-alkyl, C₂H₄SO₃-
IIIe) K = L = H and M = N and is selected from the group consisting of: 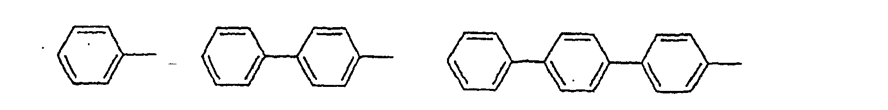 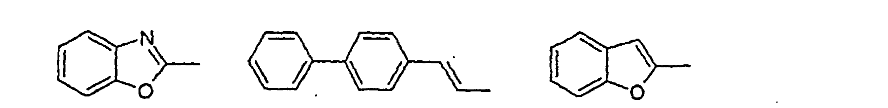 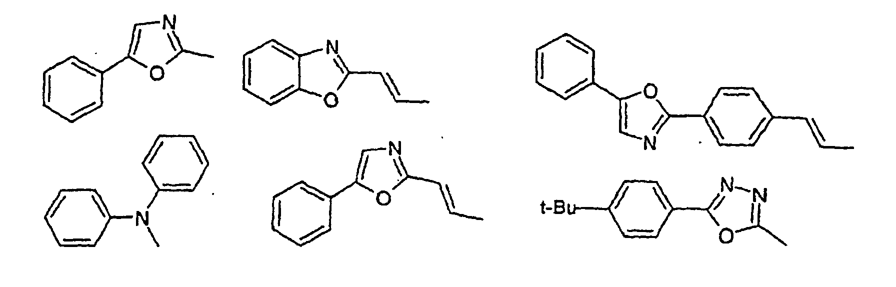
IIIf) K = L and is selected from the group consisting of: 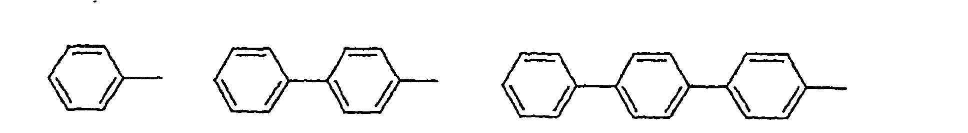 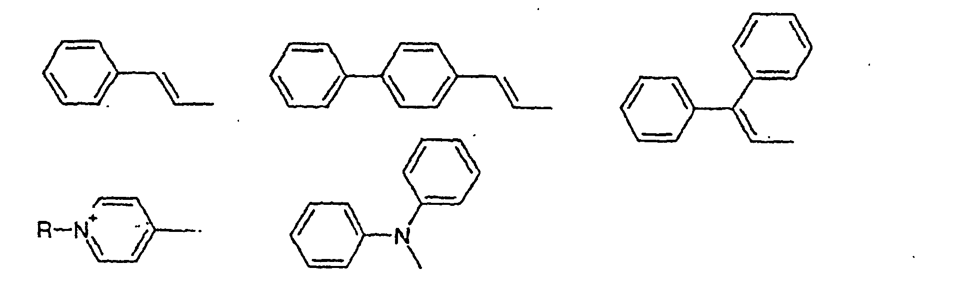 R = C₁-C₂₂-alkyl, C₂H₄SO₃-
and M = N and is selected from the group consisting of: 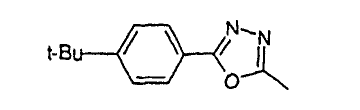 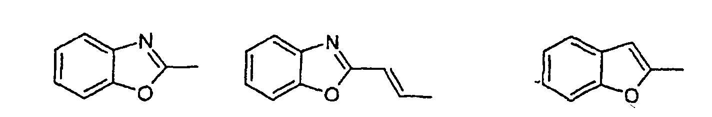 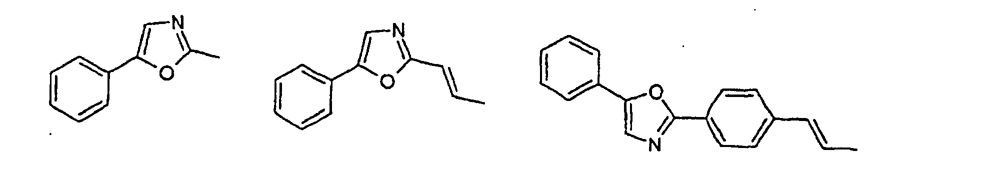
IIIg) K = L and is selected from the group consisting of: 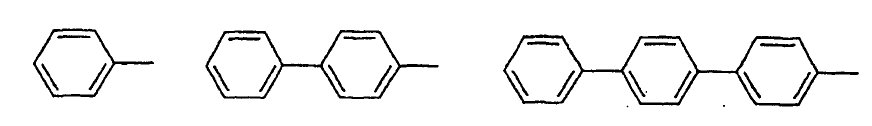 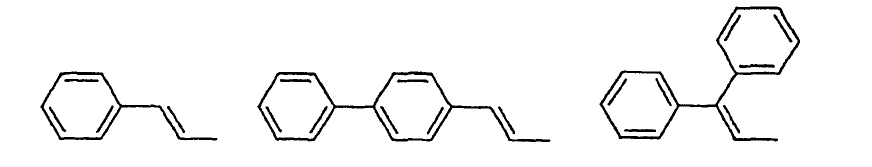 and M = N and is selected from the group consisting of: 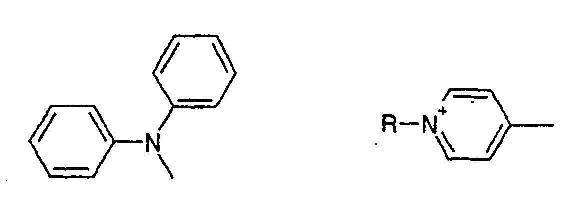 R = C₁-C₂₂-alkyl, C₂H₄SO₃-.

4. A spiro compound of the formula (V) 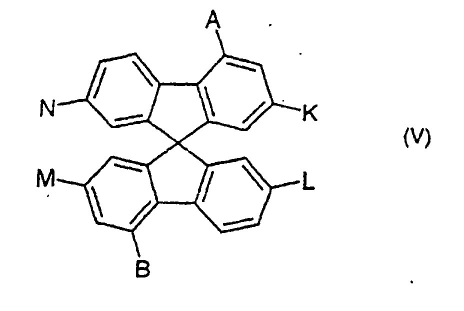 where the symbols have the following meanings: A, B are identical or different and are each 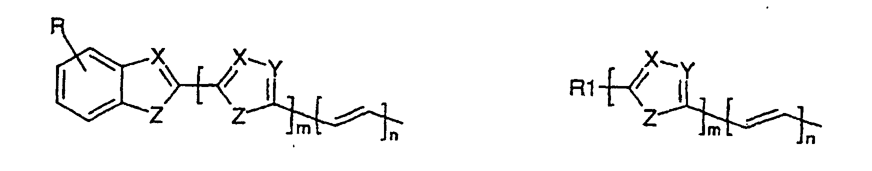 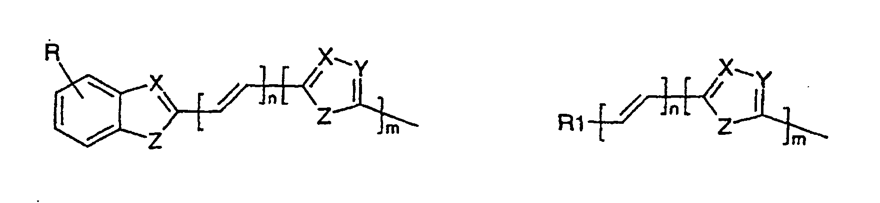 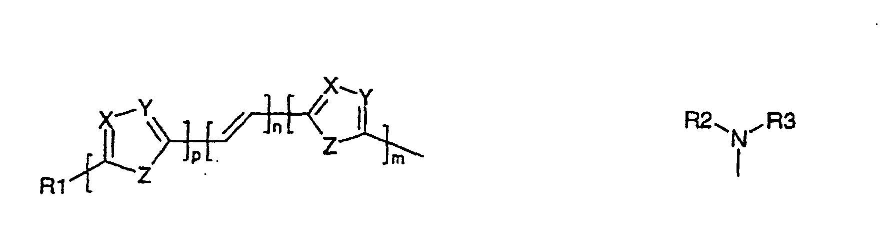 and A, B can also be identical or different and each be a linear or branched alkyl, alkyloxy or ester group having from 1 to 22 carbon atoms, -CN, -NO₂, -Ar or -O-Ar;
K, L, M, N are identical or different and are each 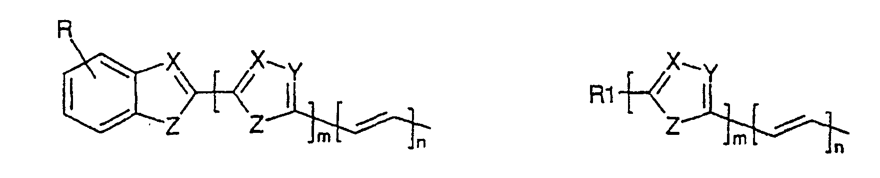 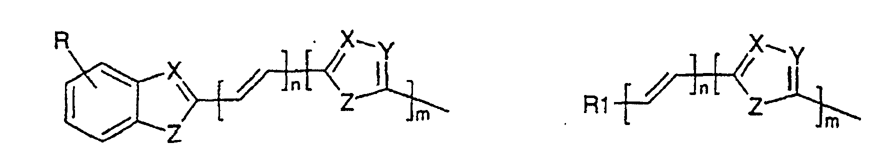 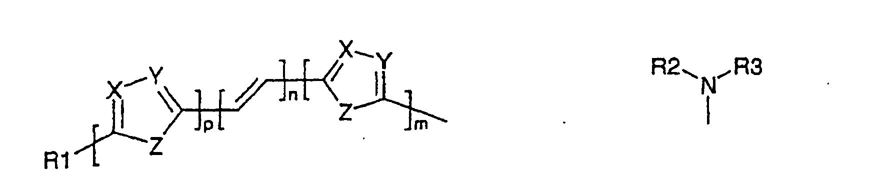 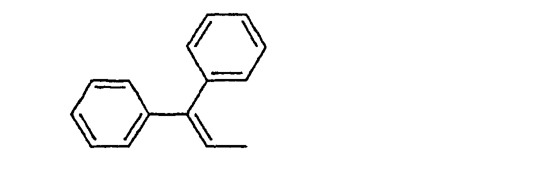
R is H, a linear or branched alkyl, alkoxy or ester group having from 1 to 22 carbon atoms, -CN, -NO₂, -NR²R³, -Ar or -O-Ar;
Ar is phenyl, biphenyl, 1-naphthyl, 2-napthyl, 2-thienyl, 2-furanyl, with each of these groups being able to bear one or two radicals R,
m, n, p are 0, 1, 2 or 3;
X, Y are identical or different and are CR or nitrogen;
Z is -O-, -S-, -NR¹-, -CR¹R⁴-, -CH=CH-, -CH=N-;
R¹ is a linear or branched alkyl, alkoxy or ester group having from 1 to 22 carbon atoms,-CN, -NO2, -NR²R³, -Ar or -O-Ar;
R⁴ is H, a linear or branched alkyl, alkoxy or ester group having from 1 to 22 carbon atoms, -CN, -NO₂, -NR²R³, -Ar or -O-Ar;
R², R³ are identical or different and are a linear or branched alkyl group having from 1 to 22 carbon atoms, -Ar or 3-methylphenyl.

5. A spiro compound of the formula

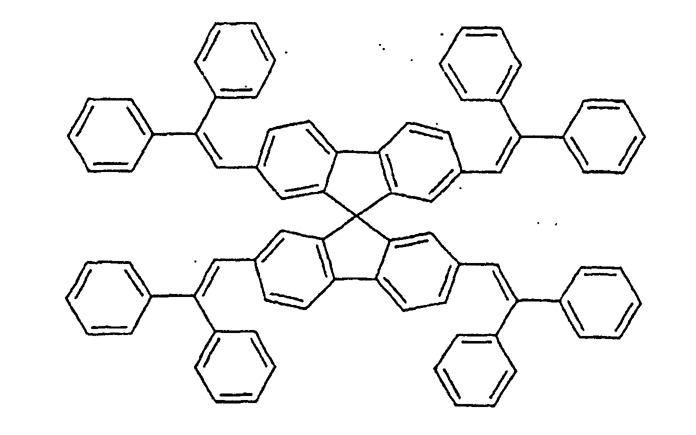

6. A spiro compound of the formula

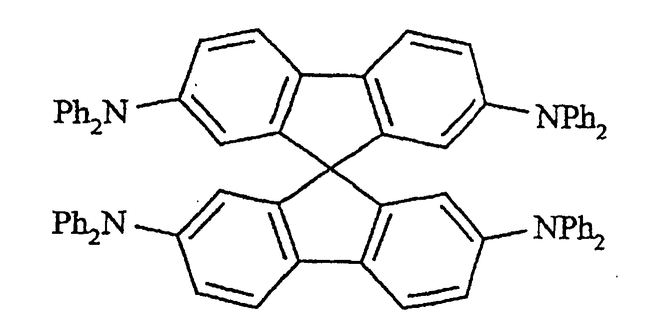

7. Use as claimed in one or more of claims 1 to 3, wherein the spiro compound serves as a light-emitting layer.

8. Use as claimed in one or more of claims 1 to 3, wherein the spiro compound serves as a transport layer.

9. Use as claimed in one or more of claims 1 to 3, wherein the spiro compound serves for charge injection.

10. An electroluminescence device comprising an active layer which comprises one or more compounds of the formula (II) to (III) as claimed in one or more of claims 1 to 3.

11. The electroluminescence device as claimed in claim 10, wherein the active layer is a light-emitting layer.

12. The electroluminescence device as claimed in claim 10, wherein the active layer is a transport layer.

13. The electroluminescence device as claimed in claim 10, wherein the active layer is a charge-injection layer.

## Revendications

1. Utilisation de composés spiraniques de formule générale (II),
dans laquelle les groupes benzoïques indépendamment les uns des autres peuvent être substitués et/ou fusionnés 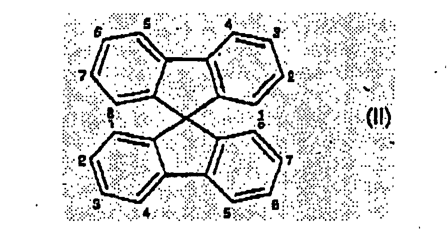 dans des dispositifs électroluminescents.

2. Utilisation selon la revendication 1, **caractérisée en ce qu'**on utilise un dérivé spirobifluorène de formule (III), 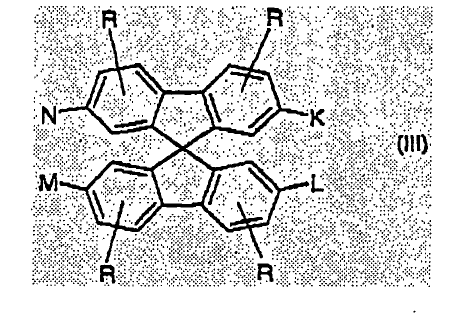 dans laquelle les symboles et indices ont les significations suivantes :
K, L, M, N sont, identiques ou différents, 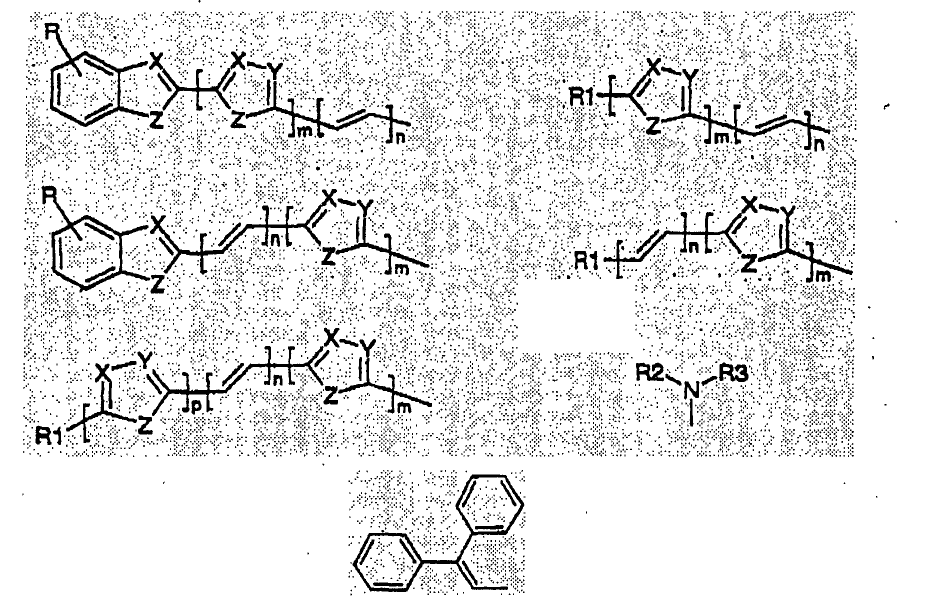
R peuvent, identiques ou différents, avoir des significations identiques à K, L, M, N ou est H, un groupe alkyle, alcoxy ou ester, linéaire ou ramifié, ayant de 1 à 22 atomes de carbone, -CN, -NO₂, -NR²R³, -Ar ou -O-Ar,
Ar est un groupe phényle, biphényle, 1-naphtyle, 2-naphtyle, 2-thiényle, 2-furanyle, dans lequel chacun de ces groupes peut porter un ou deux résidus R,
m, n, p sont 0, 1, 2 ou 3 ;
X, Y sont identiques ou différents CR, N ;
Z est -O-, -S-, -NR¹-, -CR¹R⁴-, -CH=CH-, -CH=N- ;
R¹, R⁴ peuvent, identiques ou différents, avoir la même signification que R ;
R², R³ sont identiques ou différents H, un groupe alkyle linéaire ou ramifié ayant de 1 à 22 atomes de carbone, -Ar, 3-méthylphényle.

3. Utilisation selon la revendication 2, **caractérisée en ce qu'**on utilise un dérivé spirobifluorène de formule (IIIa) à (IIIg),
IIIa) K = L = M = N et est choisi parmi : 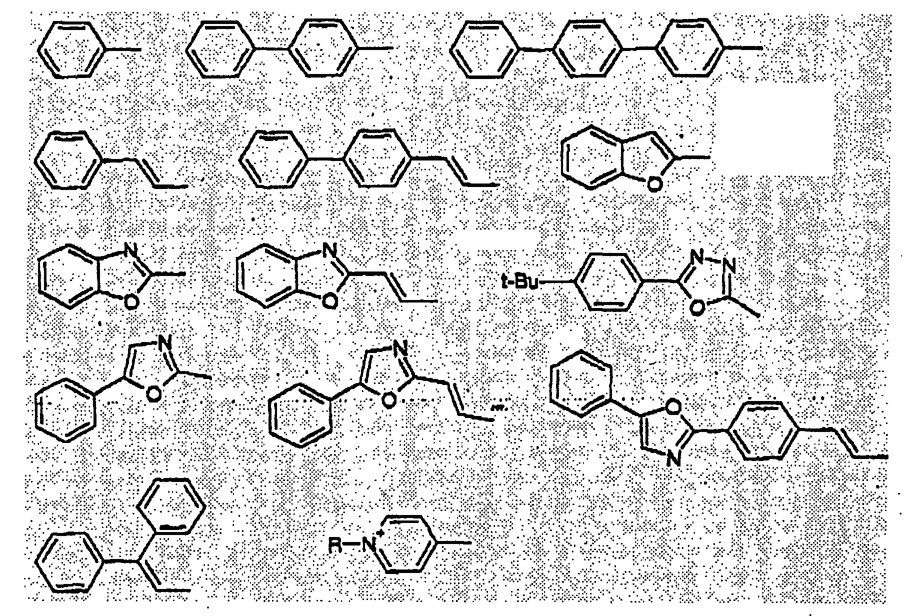 R = alkyle en C₁-C₂₂, C₂H₄SO₃-
IIIb) K = M = H et N = L et est choisi parmi : 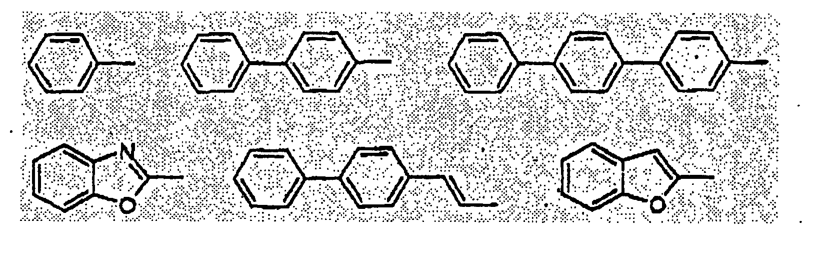 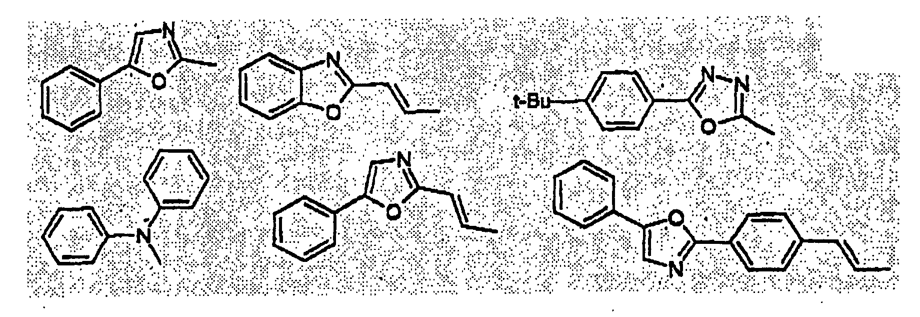
IIIc) K = M et est choisi parmi : 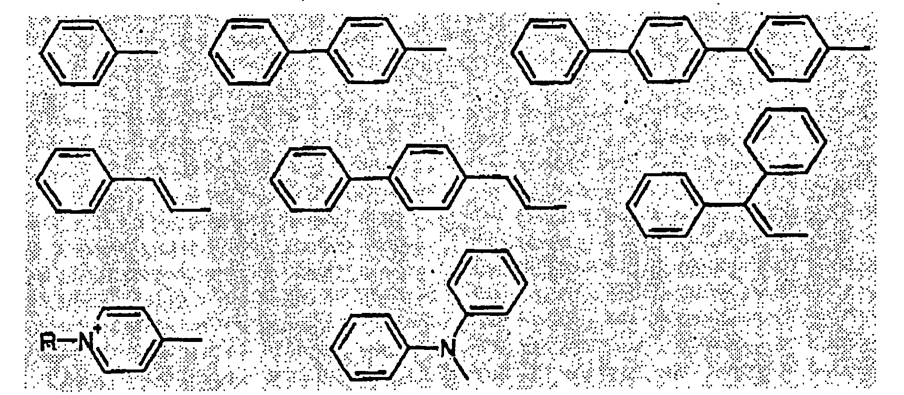
R = alkyle en C₁-C₂₂, C₂H₄SO₃-
et N = L et est choisi parmi: 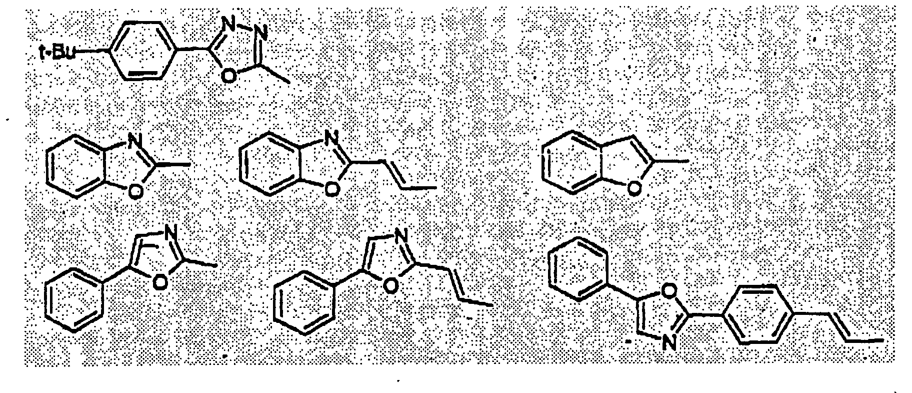
IIId) K = M et est choisi parmi : 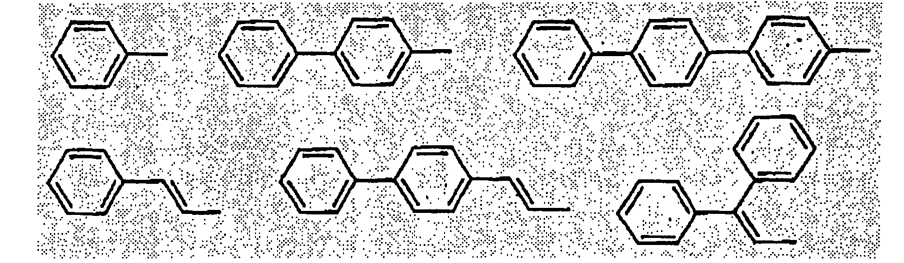 et N = L et est choisi parmi 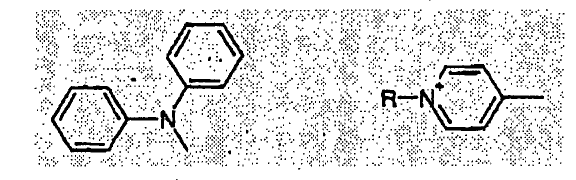 R = alkyle en C₁-C₂₂, C₂H₄SO₃-
IIIe) K = L = H et M = N et est choisi parmi : 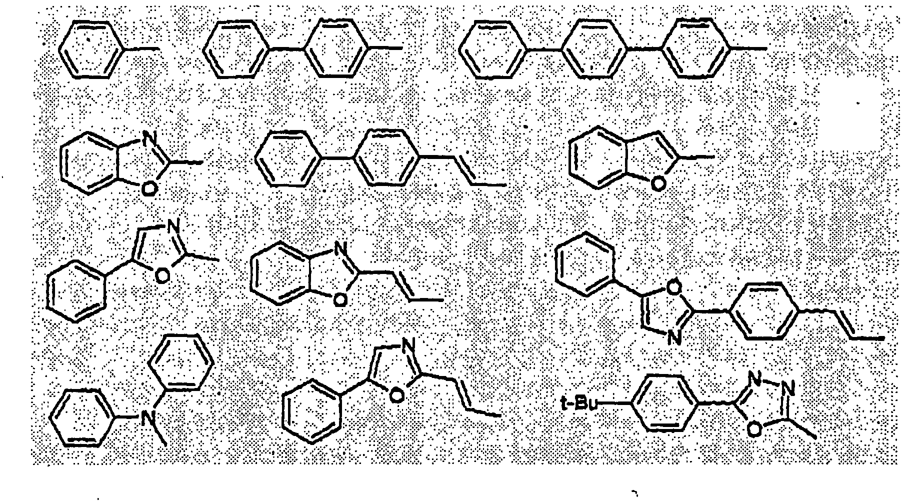
IIIf) K = L et est choisi parmi : 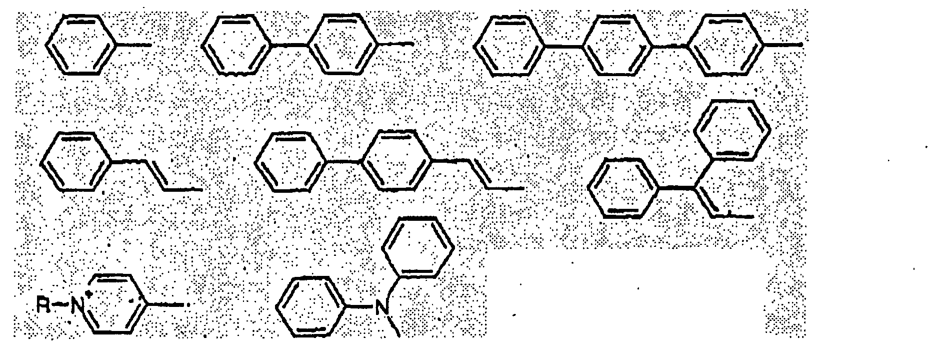 R = alkyle en C₁-C₂₂, C₂H₄SO₃-
et M = N et est choisi parmi : 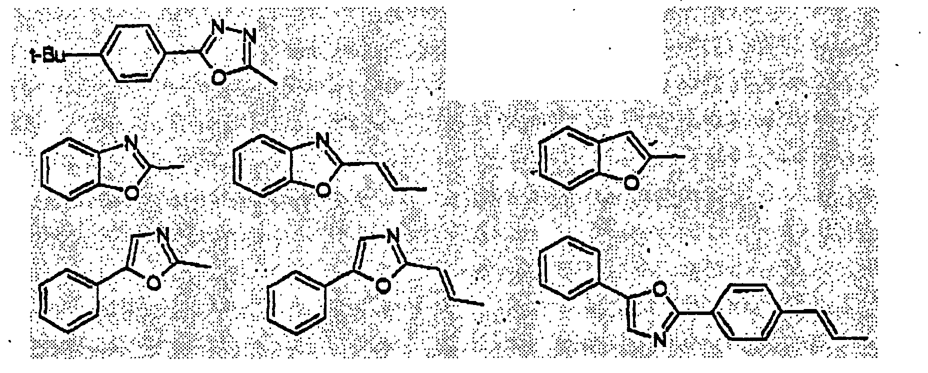
IIIg) K = L et est choisi parmi : 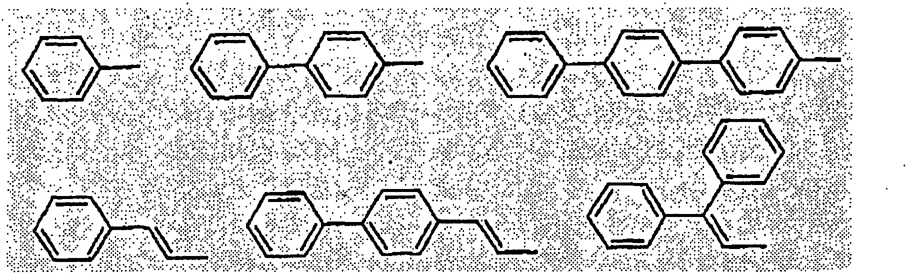 et M = N et est choisi parmi : 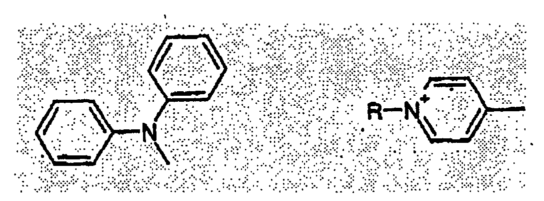 R = alkyle en C₁-C₂₂, C₂H₄SO₃-.

4. Composé spiranique de formule (V), 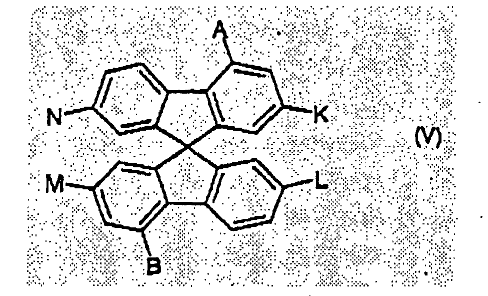 dans laquelle les symboles ont les significations suivantes :
A, B sont identiques ou différents 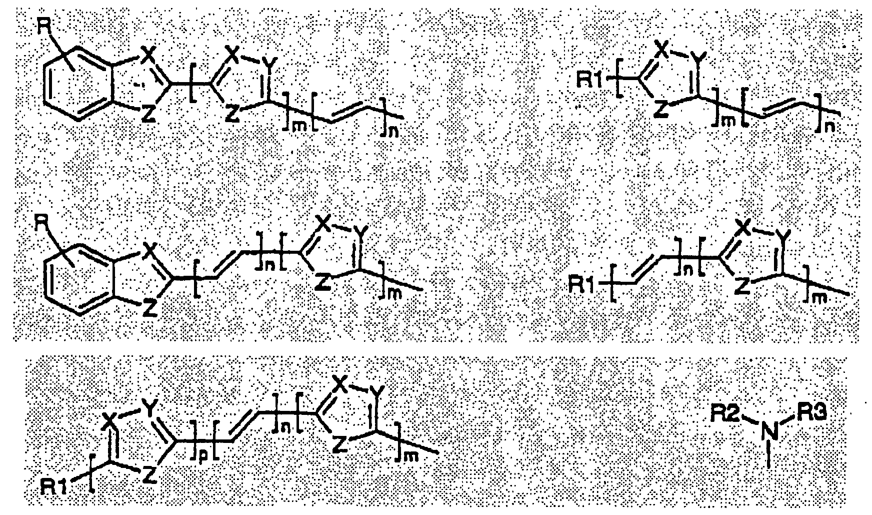
et
A, B peuvent aussi être, identiques ou différents, un groupe alkyle, alkyloxy ou ester, linéaire ou ramifié, ayant de 1 à 22 atomes de carbone, -CN, -NO₂, -Ar ou -O-Ar ;
K, L, M, N sont, identiques ou différents 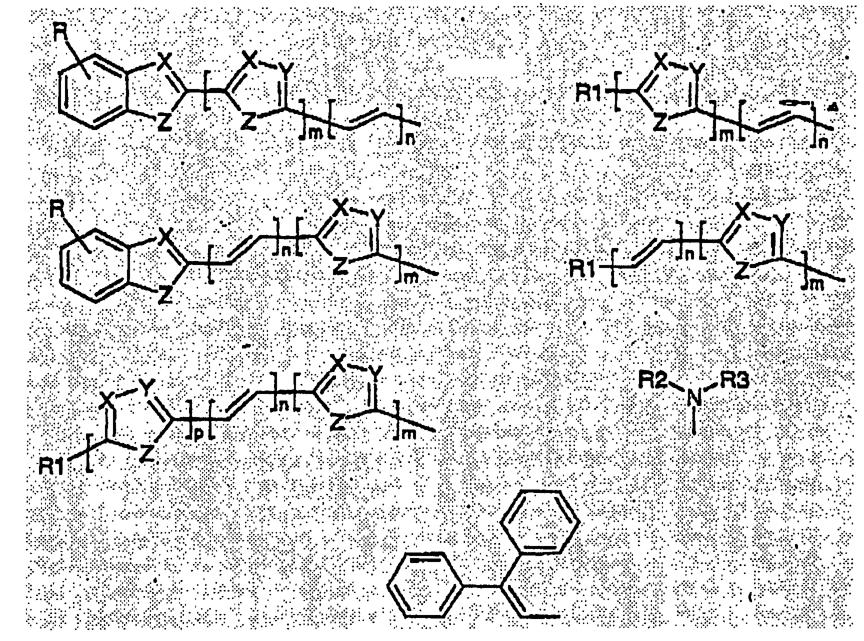
R est H, un groupe alkyle, alcoxy ou ester, linéaire ou ramifié, ayant de 1 à 22 atomes de carbone, -CN, -NO₂, -NR²R³, -Ar ou -O-Ar ;
Ar est un groupe phényle, biphényle, 1-naphtyle, 2-naphtyle, 2-thiényle, 2-furanyle, dans lequel chacun de ces groupes peut porter un ou deux résidus R,
m, n, p sont 0, 1, 2 ou 3 ;
X, Y sont identiques ou différents CR, N ;
Z est -O-, -S-, -NR¹-, -CR¹R⁴-, -CH=CH-, -CH=N- ;
R¹ est un groupe alkyle, alcoxy ou ester, linéaire ou ramifié, ayant de 1 à 22 atomes de carbone, -CN, -NO₂, -NR²R³, -Ar ou -O-Ar ;
R⁴ est H, un groupe alkyle, alcoxy ou ester, linéaire ou ramifié, ayant de 1 à 22 atomes de carbone, -CN, -NO₂, -NR²R³, -Ar ou -O-Ar ;
R², R³ sont, identiques ou différents, un groupe alkyle linéaire ou ramifié ayant de 1 à 22 atomes de carbone, -Ar ou 3-méthylphényle.

5. Composé spiranique de formule

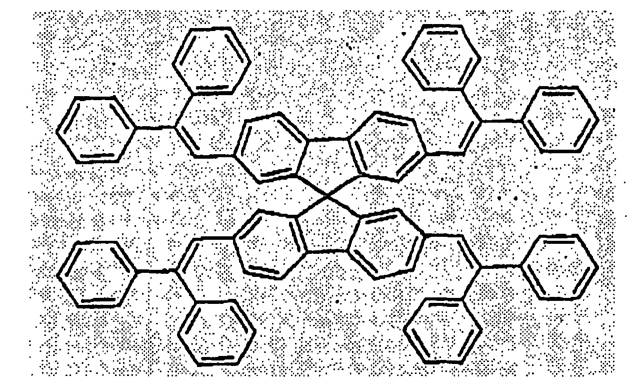

6. Composé spiranique de formule

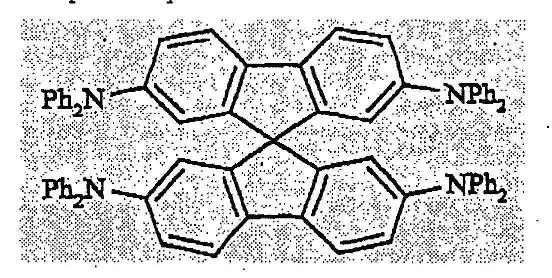

7. Utilisation selon une ou plusieurs des revendications 1 à 3, **caractérisée en ce que** le composé spiranique sert de couche photoémettrice.

8. Utilisation selon une ou plusieurs des revendications 1 à 3, **caractérisée en ce que** le composé spiranique sert de couche de transport.

9. Utilisation selon une ou plusieurs des revendications 1 à 3, **caractérisée en ce que** le composé spiranique sert à l'injection de charge.

10. Dispositif électroluminescent, contenant une couche active, qui contient un ou plusieurs composés de formule (II) à (III) selon une ou plusieurs des revendications 1 à 3.

11. Dispositif électroluminescent selon la revendication 10, **caractérisé en ce que** la couche active est une couche photoémettrice.

12. Dispositif électroluminescent selon la revendication 10, **caractérisé en ce que** la couche active est une couche de transport.

13. Dispositif électroluminescent selon la revendication 10, **caractérisé en ce que** la couche active est une couche d'injection de charge.
